(19) Europäisches Patentamt
European Patent Office
Office européen des brevets

(11) Veröffentlichungsnummer: **0 114 567 B1**

(12) **EUROPÄISCHE PATENTSCHRIFT**

(45) Veröffentlichungstag der Patentschrift: **14.09.88**

(51) Int. Cl.⁴: **C 07 D 249/08**, C 07 D 233/60, A 01 N 43/653, A 01 N 43/50, C 07 D 303/32

(21) Anmeldenummer: **83810531.0**

(22) Anmeldetag: **17.11.83**

(54) **1-Carbonyl-1-phenyl-2-azolyl-ethanol Derivate als Mikrobizide und Wuchsregulatoren, sowie ihre Zwischenprodukte.**

(30) Priorität: **23.11.82 CH 6822/82**

(43) Veröffentlichungstag der Anmeldung: **01.08.84 Patentblatt 84/31**

(45) Bekanntmachung des Hinweises auf die Patenterteilung: **14.09.88 Patentblatt 88/37**

(84) Benannte Vertragsstaaten: **AT BE CH DE FR GB IT LI LU NL SE**

(56) Entgegenhaltungen:
**EP - A - 0 078 594**
**DE - A - 2 348 663**

**CHEMICAL ABSTRACTS, Band 82, Nr. 3, 20. Januar 1975, Seite 463, Nr. 16419x, Columbus, Ohio, USA A.T. BADEV et al.: "Air oxidation of alpha-methylstyrene in liquid phase in the presence of boron promoters"**
**CHEMISCHE BERICHTE, Band 108, 1975, Seiten 2391-2396, Verlag Chemie, Weinheim, DE. M. BECKER et al.: "Synthese von Bis-epoxiden aus Dicarbonylverbindungen und Methylenbromid/Butyllithium"**
**BULLETIN DE LA SOCIETE CHIMIQUE DE FRANCE, Nr. 4, 1967, Seite 1439 J.L. PIERRE et al.: "Synthèse nouvelle d'époxydes alpha-fontionnels"**

(73) Patentinhaber: **CIBA-GEIGY AG, Klybeckstrasse 141, CH-4002 Basel (CH)**

(72) Erfinder: **Kunz, Walter, Dr., Buchenstrasse 9, CH-4104 Oberwil (CH)**
Erfinder: **Sturm, Elmar, Dr., Klusstrasse 66, CH-4147 Aesch (CH)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).



ACTORUM AG

**Beschreibung**

Die vorliegende Erfindung betrifft neue 1-Carbonyl-1-phenyl-2-azolyl-ethanol-Derivate der nachstehenden Formel I, deren Säureadditionssalze, quaternäre Azoliumsalze und Metallkomplexe. Die Erfindung betrifft ferner die Herstellung dieser Substanzen sowie mikrobizide und wuchsregulierende Mittel, die als Wirkstoff mindestens eine dieser Verbindungen enthalten. Die Erfindung betrifft auch die Herstellung der genannten Mittel und die Verwendung der Wirkstoffe oder der Mittel zur Regulierung des Pflanzenwachstums und zur Bekämpfung von schädlichen Mikroorganismen.

Die neuen Verbindungen gehorchen der Formel I

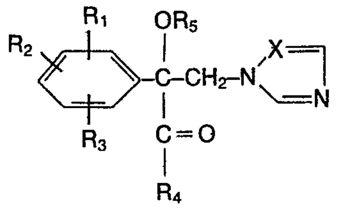

(I)

worin

$R_1$, $R_2$ und $R_3$ unabhängig voneinander für Wasserstoff, Halogen, $C_1$–$C_4$-Alkyl, $CF_3$, Phenyl oder durch $C_1$–$C_4$-Alkyl, $C_1$–$C_4$-Alkoxy, $C_1$–$C_3$-Haloalkyl, Halogen und/oder Cyano substituiertes Phenyl oder Phenoxy oder durch $C_1$–$C_4$-Alkyl, $C_1$–$C_4$-Alkoxy, $C_1$–$C_3$-Haloalkyl, Halogen und/oder Cyano substituiertes Phenoxy stehen;

$R_4$ Wasserstoff, $C_1$–$C_{10}$-Alkyl, $C_3$–$C_6$-Cycloalkyl, Phenyl oder durch $C_1$–$C_4$-Alkyl, $C_1$–$C_4$-Alkoxy, $C_1$–$C_3$-Haloalkyl, Halogen und/oder Cyano substituiertes Phenyl bedeutet;

X für –CH= oder –N= steht; und

$R_5$ Wasserstoff, $C_1$–$C_{12}$-Alkyl, $C_2$–$C_4$-Alkenyl, $C_2$–$C_4$-Alkinyl, Benzyl oder durch $C_1$–$C_4$-Alkyl, $C_1$–$C_4$-Alkoxy, $C_1$–$C_3$-Haloalkyl, Halogen und/oder Cyano substituiertes Benzyl bedeutet; unter Einschluss ihrer Säureadditionssalze, quaternären Azoliumsalze und Metallkomplexe.

Unter dem Begriff Alkyl selbst oder als Bestandteil eines anderen Substituenten sind je nach Zahl der angegebenen Kohlenstoffatome beispielsweise folgende Gruppen zu verstehen: Methyl, Ethyl, Propyl, Butyl, Pentyl, Hexyl, Heptyl, Octyl, Nonyl, Decyl usw. sowie ihre Isomeren, wie z.B. Isopropyl, Isobutyl, tert.-Butyl, Isopentyl.

Unter Halogen soll hier und im folgenden Fluor, Chlor, Brom oder Jod, vorzugsweise Fluor, Chlor oder Brom, verstanden werden.

Alkenyl bedeutet z.B. Propenyl-(1), Allyl, Butenyl-(1), Butenyl-(2) oder Butenyl-(3), Alkinyl steht z.B. für Propionyl-(1) oder Propargyl. Cycloalkyl bedeutet je nach Zahl der Kohlenstoffatome z.B. Cyclopropyl, Cyclobutyl, Cyclopentyl, Cyclohexyl, Cycloheptyl, Cyclooctyl usw. Als Substituenten für ein substituiertes Phenyl, Phenoxy oder Benzyl kommen, unabhängig von der Stellung des Phenyls, Phenoxys oder Benzyls im Molekül, folgende Substituenten in Frage: $C_1$–$C_4$-Alkyl, $C_1$–$C_4$-Alkoxy, $C_1$–$C_3$-Haloalkyl, Halogen und/oder Cyano in Frage, dabei steht Haloalkyl für einen einfach bis perhalogenierten Alkylsubstituenten, wie z.B. $CHCl_2$, $CHF_2$, $CH_2Cl$, $CCl_3$, $CH_2F$, $CH_2CH_2Cl$, $CHBr_2$, bevorzugt $CF_3$.

Die vorliegende Erfindung betrifft somit die freien organischen Verbindungen der Formel I in Form von Aldehyden und Ketonen sowie deren Säureadditionssalze, quaternäre Azoliumsalze und Metallkomplexe. Die freien Verbindungen sind bevorzugt, insbesondere die 1H,1,2,4-Triazole.

Beispiele salzbildender Säuren sind anorganische Säuren: Halogenwasserstoffsäure wie Fluorwasserstoffsäure, Chlorwasserstoffsäure, Bromwasserstoffsäure oder Jodwasserstoffsäure sowie Schwefelsäure, Phosphorsäure, phosphorige Säure, Salpetersäure und organische Säuren wie Essigsäure, Trifluoressigsäure, Trichloressigsäure, Propionsäure, Clycolsäure, Thiocyansäure, Milchsäure, Bernsteinsäure, Zitronensäure, Benzoesäure, Zimtsäure, Oxalsäure, Ameisensäure, Benzolsulfonsäure, p-Toluolsulfonsäure, Methansulfonsäure, Salicylsäure, p-Aminosalicylsäure, 2-Phenoxybenzoesäure oder 2-Acetoxybenzoesäure.

Metallkomplexe der Formel I bestehen aus dem zugrundeliegenden organischen Molekül und einem anorganischen oder organischen Metallsalz, beispielsweise den Halogeniden, Nitraten, Sulfaten, Phosphaten, Acetaten, Trifluoracetaten, Trichloracetaten, Propionaten, Tartraten, Sulfonaten, Salicylaten, Benzoaten usw. der Elemente der dritten und vierten Hauptgruppe wie Aluminium, Zinn oder Blei sowie der ersten bis achten Nebengruppe wie Chrom, Mangan, Eisen, Kobalt, Nickel, Kupfer, Zink, Silber, Quecksilber usw. Bevorzugt sind die Nebengruppen-Elemente der 4. Periode. Die Metalle können dabei in den verschiedenen ihnen zukommenden Wertigkeiten vorliegen. Die Metallkomplexe der Formel I können ein- oder mehrkernig auftreten, d.h. sie können ein oder mehrere organische Molekülanteile als Liganden erhalten. Komplexe mit den Metallen Kupfer, Zink, Mangan und Zinn sind bevorzugt.

Die Verbindungen der Formel I sind bei Raumtemperatur stabile Öle, Harze oder überwiegend Feststoffe, die sich durch sehr wertvolle mikrobizide und wuchsregulierende Eigenschaften auszeichnen. Sie lassen sich auf dem Agrarsektor oder verwandten Gebieten präventiv und kurativ zur Bekämpfung von pflanzenschädigenden Mikroorganismen und zur Regulierung des Pflanzenwuchses einsetzen, dabei sind die 1,2,4-Triazolyl(1)methylderivate im Umfang der Formel I bevorzugt. Die erfindungsgemässen Wirkstoffe der Formel I zeichnen sich durch eine sehr gute Verträglichkeit bei Kulturpflanzen aus. Ferner eignen sie sich vortrefflich zur Herstellung von Mikrobiziden und Wuchsregulatoren. Aufgrund ihrer ausgeprägten mikrobiziden und/oder wuchsregulierenden Wirkung sind diejenigen Wirksubstanzen der Formel I zunehmend bevorzugt, die folgende Substituententypen oder Kombinationen dieser Substituententypen untereinander aufweisen:

Bei $R_1$, $R_2$, $R_3$ unabhängig voneinander:

a) H, Halogen, $C_1$–$C_3$-Alkyl, $CF_3$, Phenyl, Phen-

oxy, durch $C_1$–$C_4$-Alkyl, $C_1$–$C_4$-Alkoxy, $C_1$–$C_3$-Haloalkyl, Halogen oder Cyano substituiertes Phenyl oder Phenoxy.

b) H, F, Cl, Br, $CH_3$, $C_2H_5$, $CF_3$, Phenyl, Phenoxy, $C_6H_4Cl(4)$, $C_6H_4CH_3(4)$, $C_6H_3Cl_2(2,4)$, $OC_6H_4Cl(4)$, $OC_6H_4CH_3(4)$, $OC_6H_3Cl_2(2,4)$.

c) $R_1$: H, 2-Cl, 2-Br, 2-F, 2-$CF_3$, 2-$C_6H_5$, 2-$OC_6H_5$, 3-F, 3-Cl, 3-Br, 3-$CF_2$, 3-$C_6H_5$, 3-$OC_6H_5$.

$R_2$: H, 4-Cl, 4-Br, 4-F, 4-$CF_3$, 4-$C_6H_5$, 4-$OC_6H_5$, 4-$C_6H_4Cl(4)$, 4-$C_6H_4CH_3(4)$, 4-$C_6H_3Cl_2(2,4)$, 4-$OC_6H_4Cl(4)$, 4-$OC_6H_4CH_3(4)$, 4-$OC_6H_3Cl_2(2,4)$, 4-O-$C_6H_4F(4)$, 5-Cl, 5-Br, 5-F, 5-$CF_3$.

$R_3$: H, 6-F, 6-Cl, 6-Br, 6-$CF_3$.

d) $R_1$: 2-H, 2-F, 2-Cl, 2-Br,

$R_2$: 4-F, 4-Cl, 4-Br, 4-$CF_3$

$R_3$: H.

Bei $R_4$: a) H, $C_1$–$C_6$-Alkyl, $C_3$–$C_6$-Cycloalkyl, Phenyl, durch $C_1$–$C_4$-Alkyl, $C_1$–$C_4$-Alkoxy, $C_1$–$C_3$-Haloalkyl, Halogen oder Cyano substituiertes Phenyl.

b) H, $C_1$–$C_4$-Alkyl, $C_3$–$C_6$-Cyclohexyl, Phenyl, durch Methyl, Methoxy, $CF_3$, F, Cl, Br oder CN substituiertes Phenyl.

c) H, Methyl, Phenyl, 2,4-Dichlorphenyl.

Bei $R_5$: a) Wasserstoff, $C_1$–$C_8$-Alkyl, $C_2$–$C_4$-Alkenyl, Propargyl, Benzyl, ein- oder zweifach durch Fluor, Chlor, Brom und/oder $C_1$–$C_3$-Alkyl substituiertes Benzyl.

b) Wasserstoff, $C_1$–$C_6$-Alkyl, $C_3$–$C_4$-Alkenyl, Propargyl, Benzyl, ein- oder zweifach durch Fluor, Chlor und/oder Methyl substituiertes Benzyl.

c) Wasserstoff, $C_1$–$C_5$-Allyl, Alkyl, Propargyl, Benzyl, 2-Halobenzyl, 4-Halobenzyl, 2,4-Dihalobenzyl, 2,6-Dihalobenzyl, 3,4-Dihalobenzyl, wobei Halo für Halogen steht.

d) Wasserstoff, $C_1$–$C_5$-Alkyl, Allyl, Propargyl, Benzyl, 2,6-Dichlorbenzyl, 4-Chlorbenzyl, 4-Fluorbenzyl, 2,4-Dichlorbenzyl.

Bei X: a) –CH=, –N=.

b) –N=.

Es ergeben sich daraus z.B. folgende Gruppen von Verbindungen mit steigender Bevorzugung:

a) Verbindungen der Formel I, worin $R_1$, $R_2$ und $R_3$ unabhängig voneinander für Wasserstoff, Halogen, $C_1$–$C_3$-Alkyl, $CF_3$, Phenyl, Phenoxy oder durch $C_1$–$C_4$-Alkyl, $C_1$–$C_4$-Alkoxy, $C_1$–$C_3$-Haloalkyl, Halogen oder Cyano substituiertes Phenyl oder Phenoxy stehen;

$R_4$ Wasserstoff, $C_1$–$C_6$-Alkyl, $C_3$–$C_6$-Cycloalkyl, Phenyl oder durch $C_1$–$C_4$-Alkyl, $C_1$–$C_4$-Alkoxy, $C_1$–$C_3$-Haloalkyl, Halogen oder Cyano substituiertes Phenyl bedeutet; $R_5$ für Wasserstoff, $C_1$–$C_8$-Alkyl, $C_2$–$C_4$-Alkenyl, Propargyl, Benzyl oder ein- oder zweifach durch Fluor, Chlor, Brom und/oder $C_1$–$C_3$-Alkyl substituiertes Benzyl steht und X für –CH= oder –N= steht; unter Einschluss ihrer Säureadditionssalze, quaternären Azoliumsalze und Metallkomplexe.

b) Verbindungen der Formel I, worin $R_1$, $R_2$ und $R_3$ unabhängig voneinander für Wasserstoff, Fluor, Chlor, Brom, Methyl, Ethyl, $CF_3$, Phenyl, Phenoxy, $C_6H_4Cl(4)$, $C_6H_4CH_3(4)$, $C_6H_3Cl_2(2,4)$, $OC_6H_4Cl(4)$, $OC_6H_4CH_3(4)$ oder $OC_6H_3Cl_2(2,4)$ steht;

$R_4$ Wasserstoff, $C_1$–$C_4$-Alkyl, $C_3$–$C_6$-Cycloalkyl, Phenyl oder durch Methyl, Methoxy, $CF_3$, F, Cl, Br oder CN substituiertes Phenyl bedeutet; $R_5$ für Wasserstoff, $C_1$–$C_6$-Alkyl, $C_3$–$C_4$-Alkenyl, Propargyl, Benzyl oder ein- oder zweifach durch Fluor, Chlor und/oder Methyl substituiertes Benzyl steht; und X für –N= steht; unter Einschluss ihrer Säureadditionssalze, quaternären Azoliumsalze und Metallkomplexe.

c) Verbindungen der Formel I, worin $R_1$ für Wasserstoff, 2-Cl, 2-Br, 2-F, 2-$CF_3$, 2-$C_6H_5$, 2-$OC_6H_5$, 3-F, 3-Cl, 3-Br, 3-$CF_3$, 3-$C_6H_5$ oder 3-$OC_6H_5$ steht; $R_2$ Wasserstoff, 4-Cl, 4-Br, 4-F, 4-$CF_3$, 4-$C_6H_5$, 4-$OC_6H_5$, 4-$C_6H_4Cl(4)$, 4-$C_6H_4CH_3(4)$, 4-$C_6H_3Cl_2(2,4)$, 4-$OC_6H_4Cl(4)$, 4-$OC_6H_4CH_3(4)$, 4-$OC_6H_3Cl_2(2,4)$, 4-O-$C_6H_4F(4)$, 5-Cl, 5-Br, 5-F oder 5-$CF_3$ bedeutet; $R_3$ für Wasserstoff, steht;

$R_4$ für Wasserstoff, Methyl, Phenyl oder 2,4-Dichlorphenyl steht; $R_5$ Wasserstoff, $C_1$–$C_5$-Alkyl, Allyl, Propargyl, Benzyl, 2-Halobenzyl, 4-Halobenzyl, 2,4-Dihalobenzyl, 2,6-Dihalobenzyl oder 3,4-Dihalobenzyl bedeutet und X für –N= steht; unter Einschluss der Säureadditionssalze, quaternären Azoliumsalze und Metallkomplexe.

d) Verbindungen der Formel I, worin $R_1$ für 2-H, 2-F, 2-Cl oder 2-Br steht; $R_2$ 4-F, 4-Cl, 4-Br oder 4-$CF_3$ bedeutet; $R_3$ für Wasserstoff steht;

$R_4$ für Wasserstoff, Methyl, Phenyl oder 2,4-Dichlorphenyl steht; $R_5$ Wasserstoff, $C_1$–$C_5$-Alkyl, Allyl, Propargyl, Benzyl, 2,6-Dichlorbenzyl, 4-Chlorbenzyl, 4-Fluorbenzyl oder 2,4-Dichlorbenzyl bedeutet; und X für –N= steht; unter Einschluss ihrer Säureadditionssalze, quaternären Azoliumsalze und Metallkomplexe.

Besonders bevorzugte Einzelsubstanzen sind z.B.

2-(2,4-Dichlorphenyl)-2-hydroxy-3-(1H-1,2,4-triazol-1'-yl)-propanal (Verb. Nr. 1.54);

2-(2,4-Dichlorphenyl)-2-hydroxy-1-(1H-1,2,4-triazol-1'-yl)-butan-3-on (Verb. Nr. 1.2):

2-(2,4-Dichlorphenyl)-2-hydroxy-1-(1H-1,2,4-triazol-1'-yl)-pentan-3-on (Verb. Nr. 1.6);

2-(2,4-Dichlorphenyl)-2-methoxy-1-(1H-1,2,4-triazol-1'-yl)-butan-3-on (Verb. Nr. 2.2);

2-(2-Chlor-4-fluorphenyl)-2-hydroxy-1-(1H-1,2,4-triazol-1'-yl)-butan-3-on (Verb. Nr. 1.16);

2-[2-Methyl-4-(4'-Chlorphenoxy)]-2-hydroxy-1-(1H-1,2,4-triazol-1'-yl)-butan-3-on (Verb. Nr. 1.36).

Die Verbindungen der Formel I werden dadurch hergestellt, dass man entweder ein Oxiran der Formel II

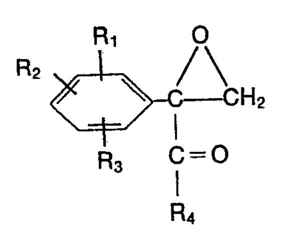

(II)

mit einem Azol der Formel III

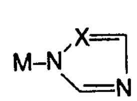

(III)

zuerst zu einer Verbindung der Formel Ia

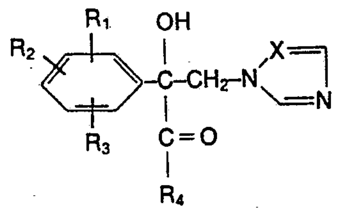

(Ia)

umsetzt, oder dadurch dass man ein α-Haloketon bzw. α-Haloaldehyd der Formel IV

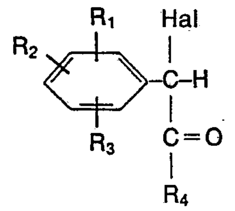

(IV)

mit Paraformaldehyd und einer starken nicht nukleophilen Base, wie z. B. einem Alkalihydrid oder Alkalialkoholat (LiH, NaH, KH, K-tert.-Butylat, $NaOC_2H_5$ usw.) reagieren lässt und durch anschliessende Zugabe eines Azols der Formel III zu einem entsprechenden Alkohol der Formel Ia weiterreagieren lässt und in beiden Fällen, sofern gewünscht, den Alkohol Ia auf übliche Weise, z. B. durch Reaktion mit einer Verbindung der Formel V

$$R_5\text{–}W \qquad (V)$$

in einen Ether der Formel I überführt, wobei die Substituenten $R_1$, $R_2$, $R_3$, $R_4$, $R_5$ und X in den Formeln Ia, II, III, IV und V die unter Formel I angegebenen Bedeutungen haben, M für Wasserstoff oder bevorzugt ein Metallatom, insbesondere ein Alkalimetallatom wie Li, Na oder K steht, Hal Halogen, insbesondere Chlor oder Brom bedeutet, und W für OH oder eine übliche Abgangsgruppe steht, dabei sollen unter einer üblichen Abgangsgruppe hier und im folgenden Substituenten wie z. B. Halogene: [wie Fluor, Chlor, Brom oder Jod, vorzugsweise Chlor oder Brom]; Sulfonyloxygruppen, bevorzugt $-OSO_2-R_a$; Acyloxygruppen, bevorzugt $-OCO-R_a$ und Isoharnstoffreste, bevorzugt

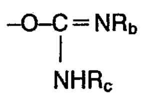

verstanden werden, wobei $R_a$, $R_b$ und $R_c$ unabhängig voneinander für $C_1$–$C_3$-Alkyl, $C_1$–$C_3$-Haloalkyl oder gegebenenfalls durch Halogen, Methyl, Nitro, Trifluormethyl und/oder Methoxy substituiertes Phenyl stehen.

Die Reaktion II mit III zu Ia wird gegebenenfalls in Gegenwart von Kondensationsmitteln oder von säurebindenden Mitteln durchgeführt. Als solche kommen organische und anorganische Basen in Betracht, z. B. tertiäre Amine wie Trialkylamine (Trimethylamin, Triethylamin, Tripropylamin usw.), Pyridin und Pyridinbasen (4-Dimethylaminopyridin, 4-Pyrrolidylaminopyridin usw.), Oxide, Hydride und Hydroxide, Carbonate und Hydrogencarbonate von Alkali- und Erdalkalimetallen (CaO, BaO, NaOH, KOH, NaH, $Ca(OH)_2$, $KHCO_3$, $NaHCO_3$, $Ca(HCO_3)_2$, $K_2CO_3$, $Na_2CO_3$), sowie Alkaliacetate wie $CH_3COONa$ oder $CH_3COOK$. Darüberhinaus eignen sich auch Alkalialkoholate wie $C_2H_5ONa$, $C_3H_7$–nONa usw. In einigen Fällen kann es von Vorteil sein, wenn man das freie Azol III (M = Wasserstoff) zuerst, z. B. in situ mit einem Alkoholat, in das entsprechende Salz überführt und anschliessend in Gegenwart einer der genannten Basen mit dem Oxiran der Formel II umsetzt. Bei der Herstellung der 1,2,4-Triazolylderivate entstehen im allgemeinen parallel auch 1,3,4-Triazolylisomere, die sich auf übliche Weise, z. B. mit unterschiedlichen Lösungsmitteln, voneinander trennen lassen.

Die Reaktion (II mit III zu Ia) wird bevorzugt in einem relativ polaren, jedoch reaktionsinerten organischen Lösungsmittel, durchgeführt, z. B. N,N-Dimethylformamid, N,N-Dimethylacetamid, Dimethylsulfoxid, Acetonitril, Benzonitril und anderen. Derartige Lösungsmittel können in Kombination mit anderen reaktionsinerten Lösungsmitteln, z. B. Benzol, Toluol, Xylol, Hexan, Petrolether, Chlorbenzol, Nitrobenzol u. a. durchgeführt werden. Die Reaktionstemperaturen liegen in einem Temperaturbereich von 0° bis 150 °C, vorzugsweise 20° bis 100 °C.

Im übrigen kann diese Reaktion (II mit III zu Ia) analog zu bereits bekannten Umsetzungen von anderen Oxiranen mit Azolen [vgl. DE-OS 2 912 288] durchgeführt werden.

Die Reaktion von Verbindungen der Formel IV zu Produkten der Formel Ia erfolgt durch Umsetzung mit Paraformaldehyd in Gegenwart einer starken, nicht nucleophilen Base, wie Alkalihydrid (NaH), und anschliessender Zugabe von Triazol oder bevorzugt dessen Alkalisalz bzw. eines Gemisches aus beiden. Die Reaktion erfolgt im allgemeinen bei Temperaturen von 0° bis +140 °C, vorzugsweise +10°, bis +80 °C und wird üblicherweise in wasserfreien Lösungsmitteln wie z. B. N,N-Dimethylformamid, N,N-Dimethylacetamid, Dimethylsulfoxid, Hexamethylphosphortriamid, N-Methylpyrrolidon, Nitrile wie Acetonitril, Ether und etherartigen Verbindungen wie Dialkylether, Tetrahydrofuran oder Dioxan usw. durchgeführt. Sie erfolgt vorzugsweise in Dimethylsulfoxid.

Bei den genannten Teilreaktionen können die Zwischenprodukte aus dem Reaktionsprodukt aus dem Reaktionsmedium isoliert und falls gewünscht, vor der Weiterreaktion, auf eine der allgemein üblichen Methoden gereinigt werden, z. B. durch Waschen, Digerieren, Extraktion, Kristallisation, Chromatographie, Destillation usw.

Die Weiterreaktion von Ia zu I erfolgt in den Fällen, in denen W in Formel V für eine übliche Abgangsgruppe steht in Abwesenheit oder bevorzugt in Anwesenheit eines reaktionsinerten Lösungsmittels. Es eignen sich z. B. folgende Lösungsmittel: N,N-Dimethylformamid, N,N-Dimethylacetamid, Hexamethylphosphortriamid, Dimethylsulfoxid, 2-Methyl-2-pentanon, usw. Auch Gemische dieser Lösungsmittel untereinander oder mit anderen üblichen inerten organischen Lösungsmitteln, z. B. mit aromatischen Kohlenwasserstoffen wie Benzol, Toluol, Xylolen usw. können verwendet werden. In manchen Fällen kann es sich als vorteilhaft erweisen, zur Beschleunigung der Reaktionsgeschwindigkeit in Gegenwart einer Base wie z.B. eines Alkalimetallhydrides, -hydroxides oder -carbonates zu arbeiten. Es kann aber auch von Vorteil sein, dass man den Alkohol der Formel Ia ($R_5$ = OH) zuerst auf an sich bekannte Weise, z.B. durch Reaktion mit einer starken Base, in ein geeignetes Metallsalz überführt.

Geeignete starke Basen sind z.B. Alkali- und Erdalkalihydride (NaH, KH, $CaH_2$ usw.) und Alkaliorganische Verbindungen wie z.B. Butyllithium oder Alkali-tert.-Butoxid, darüberhinaus können auch Alkalihydroxide, wie NaOH oder KOH eingesetzt werden, wenn man in einem wässrigen Zweiphasensystem und in Anwesenheit eines Phasentransferkatalysators arbeitet.

Man kann jedoch auch den Alkohol der Formel Ia, vor der Weiterreaktion zuerst auf übliche Weise in ein Alkalialkoholat überführen und dann mit einer Verbindung der Formel V (worin W für eine Abgangsgruppe steht) umsetzen, dabei arbeitet man vorteilhafterweise in Gegenwart eines Kronenethers. Bei M = K, insbesondere in Gegenwart von 18 Krone-6; bei M = Na, insbesondere in Gegenwart von 15 Krone-5. Dabei wird die Reaktion zweckmässigerweise in einem reaktionsinerten Medium durchgeführt. Als Lösungsmittel eignen sich z.B. Ether und etherartige Verbindungen, z.B. Diniederalkylether (Diethylether, Diisopropylether, tert.-Butylmethylether usw.), Tetrahydrofuran, Dioxan und aromatische Kohlenwasserstoffe wie Benzol, Toluol oder Xylole.

Für die organische, mit Wasser nicht mischbare Phase kommen dabei z.B. folgende Lösungsmittel in Frage: Aliphatische und aromatische Kohlenwasserstoffe, wie Pentan, Hexan, Cyclohexan, Petrolether, Ligroin, Benzol, Toluol, Xylole usw., halogenierte Kohlenwasserstoffe, wie Dichlormethan, Chloroform, Tetrachlorkohlenstoff, Ethylendichlorid, 1,2-Dichlorethan, Tetrachlorethylen usw. oder aliphatische Ether, wie Diethylether, Diisopropylether, t-Butylmethylether usw. Beispiele geeigneter Phasentransfer-Katalysatoren sind: Tetraalkylammoniumhalogenide, -hydrogensulfate oder -hydroxide wie Tetrabutylammoniumchlorid, -bromid, -jodid; Triethylbenzylammoniumchlorid, -bromid; Tetrapropylammoniumchlorid, -bromid, -jodid; usw. Als Phasentransfer-Katalysatoren kommen auch Phosphonium-Salze in Betracht. Die Reaktionstemperaturen liegen im allgemeinen zwischen 30° und 130 °C, bzw. am Siedepunkt des Lösungsmittels oder Lösungsmittelgemisches.

In den Fällen, in denen W in Formel V für Hydroxygruppen stehen, wird vorteilhafterweise eine Kondensationsreaktion durchgeführt. Beide Reaktanden werden in einem geeigneten Lösungsmittel unter Rückfluss erhitzt.

Hierbei können grundsätzlich Lösungsmittel eingesetzt werden, die sich gegenüber den Reaktionspartnern inert verhalten und zweckmässigerweise mit Wasser Azeotrope bilden. Es eignen sich hierzu beispielsweise aromatische Kohlenwasserstoffe, wie Benzol, Toluol, Xylole oder halogenierte Kohlenwasserstoffe, wie Dichlormethan, Chloroform, Tetrachlorkohlenstoff, 1,2-Dichloräthan, Tetrachloräthylen, Chlorbenzol, aber auch ätherartige Verbindungen, wie tert.-Butylmethyläther, Dioxan und andere. In manchen Fällen kann die Verbindung der Formel III selbst als Lösungsmittel verwendet werden. Bei dieser Kondensationsreaktion arbeitet man zweckmässigerweise in Gegenwart einer starken Säure, z.B. Paratoluolsulfonsäure und bei Siedetemperaturen der azeotropen Mischung.

Zur Herstellung der Ether der Formel I kann man auch die freie OH-Gruppe in den Verbindungen der Formel Ia, zuerst gegen eine der obengenannten, üblichen Abgangsgruppen W austauschen und anschliessend mit einer Verbindung der Formel V (mit W = OH) umsetzen.

Der Austausch der freien Hydroxylgruppe in den Verbindungen der Formel Ia gegen eine Abgangsgruppe W wird bevorzugt in einem reaktionsinerten Lösungsmittel durchgeführt. Beispiele solcher Lösungsmittel sind:

Aromatische und aliphatische Kohlenwasserstoffe wie Benzol, Toluol, Xylole, Petrolether, Ligroin oder Cyclohexan; halogenierte Kohlenwasserstoffe wie Chlorbenzol, Methylenchlorid, Ethylenchlorid, Chloroform, Tetrachlorkohlenstoff oder Tetrachlorethylen; Ether und etherartige Verbindungen wie Diethylether, Diisopropylether, T-Butylmethylether, Dimethoxyethan, Dioxan, Tetrahydrofuran oder Anisol; Ester wie Ethylacetat, Propylacetat oder Butylacetat; Nitrole wie Acetonitril oder Verbindungen wie Dimethylsulfoxid, Dimethylformamid und Gemische solcher Lösungsmittel untereinander.

Die Einführung der Abgangsgruppe W erfolgt nach üblichen Methoden. Bedeutet A Chlor, so wird als Reagenz z.B. Phosphoroxychlorid, Phosphortrichlorid, Phosphorpentachlorid oder vorzugsweise Thionylchlorid eingesetzt. Man arbeitet im allgemeinen bei Temperaturen von 0° bis +120 °C. Im Falle von W = Brom verwendet man bevorzugt Phosphortribromid oder Phosphorpentabromid und führt die Reaktion bei 0° bis +50 °C durch. Steht W für eine der Gruppen $-OSO_2R_a$, $-OCO-R_a$ oder

$-O-C=NR_b$ ,
$\quad\;\; |$
$\quad\; NHR_c$

so wird als Reagenz üblicherweise das entsprechende Säurechlorid bzw. Amidinochlorid eingesetzt. Hierbei ist es zweckmässig, wenn die Reaktion bei Temperaturen von −20° bis +50 °C, vorzugsweise −10° bis +30 °C, und in Gegenwart einer schwachen Base wie Pyridin oder Triethylamin durchgeführt wird.

Die Ausgangsprodukte der Formeln III und V sind allgemein bekannt oder lassen sich nach an sich bekannten Methoden herstellen.

Die Oxirane der Formel II sind mit Ausnahme der Verbindungen 2-Formyl-2-phenyl-oxiran und 2-Benzoyl-2-phenyl-oxiran neu. Die bekannten Verbindungen sind in der wissenschaftlichen Literatur [Chem. Ber. 108, 2391–2396 (1975) bzw. Bull. Soc. France 4, 1439 (1967)] als Syntheseprodukte beschrieben, jedoch ohne Angabe von biologischen Eigenschaften oder Hinweise auf biologische Aktivitäten von daraus herstellbaren Stoffen. Die neuen Verbindungen der Formel II stellen besonders entwickelte Zwischenprodukte zur Herstellung der wertvollen Wirkstoffe der Formel I dar. Aufgrund ihrer strukturellen Beschaffenheit lassen sie sich in einfacher Weise in die Verbindungen der Formel I überführen, darüber hinaus zeigen Verbindungen der Formel II zum Teil fungizide Aktivität gegenüber Schadpilzen aus den Familien Ascomycetes, Basidiomycetes oder Fungi imperfecti.

Die Oxirane der Formel II lassen sich in an sich bekannter Weise durch Epoxidierung aus den zugrundeliegenden Styrolderivaten der Formel VI,

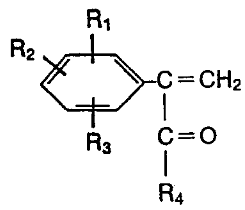

(VI)

worin $R_1$ bis $R_4$ die unter Formel I angegebenen Bedeutungen haben, beispielsweise durch Oxydation mit Persäuren wie Peressigsäure, tert.-Butyl-hydroperoxid, m-Chlorperbenzoesäure, $H_2O_2$ usw., und gegebenenfalls in Gegenwart von Basen wie NaOH, KOH, $NaHCO_3$ in gängigen reaktionsinerten Lösungsmitteln herstellen. Hierbei kann $Mo(CO)_6$ als Katalysator eingesetzt werden.

Styrolderivate der Formel VI, sind aus den entsprechenden, an sich bekannten Ausgangsketonen VII,

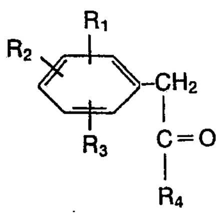

(VII)

worin $R_1$ bis $R_4$ wie unter Formel I definiert sind, analog zu Angew. Chem. 1976, 261, durch Reaktion mit Dimethylmethylenimmoniumhalogeniden zugänglich.

Die Verbindungen der Formel IV sind bekannt oder können nach an sich bekannten Methoden hergestellt werden, z.B. aus den zugrundeliegenden, bekannten α-Hydroxyketonen bzw. α-Hydroxyaldehyden durch üblichen Austausch der Hydroxygruppen gegen Halogen.

Bei der Herstellung aller hierin genannten Ausgangs-, Zwischen- und Endprodukte können grundsätzlich, sofern nicht ausdrücklich im einzelnen spezifiziert, ein oder mehrere reaktionsinerte Lösungs- oder Verdünnungsmittel anwesend sein. In Frage kommen beispielsweise aliphatische und aromatische Kohlenwasserstoffe wie Benzol, Toluol, Xylole, Petrolether; halogenierte Kohlenwasserstoffe wie Chlorbenzol, Methylenchlorid, Ethylenchlorid, Chloroform, Tetrachlorkohlenstoff, Tetrachlorethylen; Ether und etherartige Verbindungen wie Dialkylether (Diethylether, Diisopropylether, tert.-Butylmethylether usw.), Anisol, Dioxan, Tetrahydrofuran; Nitrile wie Acetonitril, Propionitril; N,N-dialkylierte Amide wie Dimethylformamid; Dimethylsulfoxid; Ketone wie Aceton, Diethylketon, Methylethylketon und Gemische solcher Lösungsmittel untereinander. In manchen Fällen kann es auch von Vorteil sein, wenn die Reaktion oder Teilschritte einer Reaktion unter Schutzgasatmosphäre und/oder absoluten Lösungsmitteln durchgeführt werden. Als Schutzgase eignen sich inerte Gase wie Stickstoff, Helium, Argon oder in gewissen Fällen auch Kohlendioxid.

Die Verbindungen der Formel I

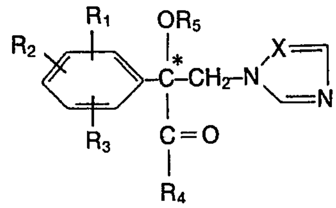

(I)

besitzen in Nachbarstellung zu den Substituenten A und $OR_5$ stets ein asymmetrisches C-Atom C* und können daher in zwei enantiomeren Formen vorliegen. Im allgemeinen entsteht bei der Herstellung dieser Substanzen ein Gemisch beider Enantiomerer, dieses lässt sich auf übliche Weise, z.B. durch fraktionierte Kristallisation von Salzen mit optisch aktiven, starken Säuren, in die reinen optischen Antipoden aufspalten. Die Enantiomeren können unterschiedlich biologische Wirkungen aufweisen, so kann z.B. bei der einen Form die fungizide und bei der anderen die pflanzenregulatorische Wirkung im Vordergrund stehen. Auch kann bei gleichem Wirkungsspektrum ein gradueller Aktivitätsunterschied auftreten. Die Acetalisierung von Ketonen der Formel I mit unsymmetrischen 1,2- oder 1,3-Diolen führt zu einem weiteren Asymmetriezentrum im resultierenden Dioxolan- bzw. Dioxanring. Es entstehen 4 Stereoisomere, die als Diastereomerenpaare vorliegen (cis- und trans-Form). Die einzelnen Diastereomeren lassen sich auf übliche Weise, z.B. durch Säulenchromatographie voneinander trennen und, sofern gewünscht, in die Enantiomeren aufspalten.

Die vorliegende Erfindung betrifft alle reinen Stereoisomeren, Enantiomeren und deren Gemische untereinander.

Das beschriebene Herstellungsverfahren ist, einschliesslich aller Teilschritte, ein wichtiger Bestandteil der vorliegenden Erfindung.

Es wurde nun überraschend gefunden, dass die neuen Wirkstoffe der Formel I bzw. Mittel, die diese Wirkstoffe enthalten, sich unter anderem allem dadurch auszeichnen, dass sie gezielt in den Metabolismus der Pflanzen eingreifen. Dieser gezielte Eingriff in die physiologischen Vorgänge der Pflanzenentwicklung macht die Wirkstoffe der Formel I für verschiedene Zwecke verwendbar,

insbesondere für solche, die mit der Ertragssteigerung bei Nutzpflanzen, der Ernteerleichterung und der Arbeitseinsparung bei Massnahmen an Pflanzenkulturen im Zusammenhang stehen. Die Verbindungen der Formel I lassen sich somit zur Regulierung des Pflanzenwachstums einsetzen.

Pflanzenwuchsregulierende Stoffe können zum Beispiel zur Hemmung des vegetativen Pflanzenwachstums eingesetzt werden.

Ein weiterer Mechanismus der Ertragssteigerung mit Wachstumshemmern beruht darauf, dass die Nährstoffe in stärkerem Masse der Blüten- und Fruchtbildung zugute kommen, während das vegetative Wachstum eingeschränkt wird.

Weiter hat es sich überraschenderweise gezeigt, dass die Aktivsubstanzen der Formel I bzw. entsprechende Mittel, ausser vorteilhaften wuchsregulierenden Eigenschaften insbesondere ein für praktische Bedürfnisse sehr günstiges Mikrobizidspektrum aufweisen. Deshalb liegt ein weiteres Einsatzgebiet von Verbindungen der Formel I in der Bekämpfung von schädlichen Mikroorganismen, vor allem von phytopathogenen Pilzen. So besitzen die Verbindungen der Formel I eine für praktische Bedürfnisse sehr günstige kurative, präventive und systemische Wirkung zum Schutz von Pflanzen, insbesondere Kulturpflanzen, ohne diese nachteilig zu beeinflussen.

Mit den Wirkstoffen der Formel I können an Pflanzen oder an Pflanzenteilen (Früchte, Blüten, Laubwerk, Stengel, Knollen, Wurzeln) von unterschiedlichen Nutzkulturen die auftretenden Mikroorganismen eingedämmt oder vernichtet werden, wobei auch später zuwachsende Pflanzenteile von derartigen Mikroorganismen verschont bleiben.

Die Wirkstoffe sind gegen die den folgenden Klassen angehörenden phytopathogenen Pilze wirksam: Ascomyceten (z.B. Venturia, Podosphaera, Erysiphe, Monilinia, Uncinula); Basidiomyceten (z.B. die Gattungen Hemileia, Rhizoctonia, Puccinia); Fungi imperfecti (z.B. Botrytis, Helminthosporium, Fusarium, Septoria, Cercospora und Alternaria). Überdies wirken die Verbindungen der Formel I systemisch. Sie können ferner als Beizmittel zur Behandlung von Saatgut (Früchte, Knollen, Körner) und Pflanzenstecklingen zum Schutz vor Pilzinfektionen sowie gegen im Erdboden auftretende phytopathogene Pilze eingesetzt werden. Die erfindungsgemässen Wirkstoffe zeichnen sich durch besonders gute Pflanzenverträglichkeit aus.

Die Erfindung betrifft somit auch mikrobizide Mittel sowie die Verwendung der Verbindungen der Formel I zur Bekämpfung phytopathogener Mikroorganismen, insbesondere pflanzenschädigender Pilze bzw. die präventive Verhütung eines Befalls an Pflanzen.

Darüberhinaus schliesst die vorliegende Erfindung auch die Herstellung agrochemischer Mittel ein, die gekennzeichnet ist durch das innige Vermischen der Aktivsubstanz mit einem oder mehrere hierin beschriebenen Substanzen bzw. Substanzgruppen. Eingeschlossen ist auch ein Verfahren zur Behandlung von Pflanzen, das sich durch Applikation der Verbindungen der Formel I bzw. der neuen Mittel auszeichnet.

Als Zielkulturen für die hierin offenbarten Indikationsgebiete gelten im Rahmen dieser Erfindung beispielsweise folgende Pflanzenarten: Getreide: (Weizen, Gerste, Roggen, Hafer, Reis, Sorhum und Verwandte); Rüben: (Zucker- und Futterrüben); Kern-, Stein- und Beerenobst: (Äpfel, Birnen, Pflaumen, Pfirsiche, Mandeln, Kirschen, Erd-, Him- und Brombeeren); Hülsenfrüchte: (Bohnen, Linsen, Erbsen, Soja); Ölkulturen: (Raps, Senf, Mohn, Oliven, Sonnenblumen, Kokos, Rizinus, Kakao, Erdnüsse); Gurkengewächse: (Kürbis, Gurken, Melonen); Fasergewächse: (Baumwolle, Flachs, Hanf, Jute); Citrusfrüchte: (Orangen, Zitronen, Pampelmusen, Mandarinen); Gemüsesorten: (Spinat, Kopfsalat, Spargel, Kohlarten, Möhren, Zwiebeln, Tomaten, Kartoffeln, Paprika); Lorbeergewächse: (Avocado, Cinnamonum, Kampfer): oder Pflanzen wie Mais, Tabak, Nüsse, Kaffee, Zuckerrohr, Tee, Weintrauben, Hopfen, Bananen- und Naturkautschukgewächse. Pflanzen seien im Rahmen vorliegender Erfindung aber auch alle Arten von sonstigen Grünbewachsungen, seien es Zierpflanzen (Compositen), Grasflächen, Böschungen oder allgemeine niedrige Bodenbedekkungen (cover crops), die einer Erosion oder Austrocknung des Bodens entgegenwirken oder Bodenbedeckungen wie sie in Baum- und Staudenkulturen (Obstplantagen, Hopfenkulturen, Maisfeldern, Weingärten usw.) erwünscht sind.

Wirkstoffe der Formel I werden im Agrarbereich üblicherweise in Form von Zusammensetzungen verwendet und können gleichzeitig oder nacheinander mit weiteren Wirkstoffen auf die zu behandelnde Fläche oder Pflanze gegeben werden. Diese weiteren Wirkstoffe können sowohl Düngemittel, Spurenelement-Vermittler oder andere das Pflanzenwachstum beeinflussende Präparate sein. Es können aber auch selektive Herbizide, Insektizide, Fungizide, Bakterizide, Nematizide, Molluskizide oder Gemische mehrerer dieser Präparate sein, zusammen mit gegebenenfalls weiteren in der Formulierungstechnik üblichen Trägerstoffen, Tensiden oder anderen applikationsfördernden Zusätzen.

Geeignete Träger und Zusätze können fest oder flüssig sein und entsprechen den in der Formulierungstechnik zweckdienlichen Stoffen, wie z.B. natürlichen oder regenerierten mineralischen Stoffen, Lösungs-, Dispergier-, Netz-, Haft-, Verdickungs-, Binde- oder Düngemitteln. Vorteilhafte Formulierungshilfsstoffe sind ferner Phospholipide.

Ein bevorzugtes Verfahren zum Aufbringen eines Wirkstoffes der Formel I bzw. eines agrochemischen Mittels, das mindestens einen dieser Wirkstoffe enthält, ist das Aufbringen auf das Blattwerk (Blattapplikation). Anzahl der Applikationen richten sich dabei nach dem Befallsdruck für den entsprechenden Erreger (Pilzsorte) oder der Art der Wachstumsbeeinflussung. Die Wirkstoffe der Formel I können aber auch über den Erdboden durch das Wurzelwerk in die Pflanzen gelangen (systemische Wirkung), indem man den Standort

der Pflanzen mit einer flüssigen Zubereitung tränkt oder die Substanzen in fester Form in den Boden einbringt, z.B. in Form von Granulat (Bodenapplikation). Die Verbindungen der Formel I können aber auch auf Samenkörner aufgebracht werden (Coating), indem man die Körner entweder mit einer flüssigen Zubereitung des Wirkstoffs tränkt oder sie mit einer festen Zubereitung beschichtet. Darüberhinaus sind in besonderen Fällen weitere Applikationsarten möglich, so z.B. die gezielte Behandlung der Pflanzenstengel oder der Knospen.

Die Verbindungen der Formel I werden dabei in unveränderter Form oder vorzugsweise zusammen mit den in der Formulierungstechnik üblichen Hilfsmitteln eingesetzt und werden daher z.B. zu Emulsionskonzentraten, streichfähigen Pasten, direkt versprühbaren oder verdünnbaren Lösungen, verdünnten Emulsionen, Spritzpulvern, löslichen Pulvern, Stäubemitteln, Granulaten, durch Verkapselungen in z.B. polymeren Stoffen in bekannter Weise verarbeitet. Die Anwendungsverfahren wie Versprühen, Vernebeln, Verstäuben, Verstreuen, Bestreichen oder Giessen werden gleich wie die Art der Mittel den angestrebten Zielen und den gegebenen Verhältnissen entsprechend gewählt. Günstige Aufwandmengen liegen im allgemeinen bei 10 g bis 5 kg Aktivsubstanz (AS) je ha; bevorzugt 100 g bis 2 kg AS/ha, insbesondere bei 200 g bis 600 g AS/ha.

Die Formulierungen d.h. die den Wirkstoff der Formel I und gegebenenfalls einen festen oder flüssigen Zusatzstoff enthaltenden Mittel, Zubereitungen oder Zusammensetzungen werden in bekannter Weise hergestellt, z.B. durch inniges Vermischen und/oder Vermahlen der Wirkstoffe mit Streckmitteln, wie z.B. mit Lösungsmitteln, festen Trägerstoffen, und gegebenenfalls oberflächenaktiven Verbindungen (Tensiden).

Als Lösungsmittel können in Frage kommen: Aromatische Kohlenwasserstoffe, bevorzugt die Fraktionen $C_8$ bis $C_{12}$, wie z.B. Xylolgemische oder substituierte Naphthaline, Phthalsäureester wie Dibutyl- oder Dioctylphthalat, aliphatische Kohlenwasserstoffe wie Cyclohexan oder Paraffine, Alkohole und Glykole sowie deren Ether und Ester, wie Ethanol, Ethylenglykol, Ethylenglykolmonomethyl- oder Ethylether, Ketone wie Cyclohexanon, stark polare Lösungsmittel wie N-Methyl-2-pyrrolidon, Dimethylsulfoxid oder Dimethylformamid, sowie gegebenenfalls epoxydierte Pflanzenöle wie epoxydiertes Kokosnussöl oder Sojaöl; oder Wasser.

Als feste Trägerstoffe, z.B. für Stäubemittel und dispergierbare Pulver, werden in der Regel natürliche Gesteinsmehle verwendet, wie Calcit, Talkum, Kaolin, Montmorillonit oder Attapulgit. Zur Verbesserung der physikalischen Eigenschaften können auch hochdisperse Kieselsäure oder hochdisperse saugfähige Polymerisate zugesetzt werden. Als gekörnte, adsorptive Granulatträger kommen poröse Typen wie z.B. Bimsstein, Ziegelbruch, Sepiolit oder Bentonit, als nicht sorptive Trägermaterialien z.B. Calcit oder Sand in Frage. Darüberhinaus kann eine Vielzahl von vorgranulierten Materialien anorganischer oder organischer Natur wie insbesondere Dolomit oder zerkleinerte Pflanzenrückstände verwendet werden. Besonders vorteilhaft können auch Phospholipide eingesetzt werden.

Als oberflächenaktive Verbindungen kommen je nach Art des zu formulierenden Wirkstoffes der Formel I nichtionogene, kation- und/oder anionaktive Tenside mit guten Emulgier-, Dispergier- und Netzeigenschaften in Betracht. Unter Tensiden sind auch Tensidgemische zu verstehen.

Geeignete anionische Tenside können sowohl sog. wasserlösliche Seifen, als auch wasserlösliche synthetische oberflächenaktive Verbindungen sein.

Als Seifen seien die Alkali-, Erdalkali- oder gegebenenfalls substituierten Ammoniumsalze von höheren Fettsäuren ($C_{10}$–$C_{22}$), wie z.B. die Na- oder K-Salze der Öl- oder Stearinsäure, oder von natürlichen Fettsäuregemischen, die z.B. aus Kokosnuss- oder Talgöl gewonnen werden können. Ferner sind auch die Fettsäure-methyllaurinsalze zu erwähnen.

Häufiger werden jedoch sog. synthetische Tenside verwendet, insbesondere Fettsulfonate, Fettsulfate, sulfonierte Benzimidazolderivate oder Alkylarylsulfonate.

Die Fettsulfonate oder -sulfate liegen in der Regel als Alkali-, Erdalkali- oder gegebenenfalls substituierte Ammoniumsalze vor und weisen einen Alkalirest mit 8 bis 22 C-Atomen auf, wobei Alkyl auch den Alkylteil von Acylresten einschliesst, z.B. das Na- oder Ca-Salz der Ligninsulfonsäure, des Dodecylschwefelsäureesters oder eines aus natürlichen Fettsäuren hergestellten Fettalkoholsulfatgemisches. Hierher gehören auch die Salze der Schwefelsäureester und Sulfonsäuren von Fettalkohol-Ethylenoxyd-Addukten. Die sulfonierten Benzimidazolderivate enthalten, vorzugsweise 2-Sulfonsäuregruppen und einen Fettsäurerest mit 8–22 C-Atomen. Alkylarylsulfonate sind z.B. die Na-, Ca- oder Triethanolaminsalze der Dodecylbenzolsulfonsäure, der Dibutylnaphthalinsulfonsäure, oder eines Naphthalinsulfonsäure-Formaldehydkondensationsproduktes.

Ferner kommen auch entsprechende Phosphate wie z.B. Salze des Phosphorsäureesters eines p-Nonylphenol-(4–14)-Ethylenoxyd-Adduktes in Frage.

Als nichtionische Tenside kommen in erster Linie Polyglykoletherderivate von aliphatischen oder cycloaliphatischen Alkoholen, gesättigten oder ungesättigten Fettsäuren und Alkylphenolen in Frage, die 3 bis 30 Glykolethergruppen von 8 bis 20 Kohlenstoffatome im (aliphatischen) Kohlenwasserstoffrest und 6 bis 18 Kohlenstoffatome im Alkylrest der Alkylphenole enthalten können.

Weitere geeignete nichtionische Tenside sind die wasserlöslichen, 20 bis 250 Äthylenglykoläthergruppen und 10 bis 100 Propylenglykolethergruppen enthaltenden Polyethylenoxidaddukte an Polypropylenglykol, Äthylendiaminopolypropylenglykol und Alkylpolypropylenglykol mit 1 bis 10 Kohlenstoffatomen in der Alkylkette. Die genannten Verbindungen enthalten üblicherweise pro

Propylenglykol-Einheit 1 bis 5 Ethylenglykoleinheiten.

Als Beispiele nichtionischer Tenside seien Nonylphenolpolyethoxyethanole, Ricinusölpolyglycolether, Polypropylen-Polyethylenoxydaddukte, Tributylphenoxypolyethoxyethanol, Polyethylenglykol und Octylphenoxypolyethoxyethanol erwähnt.

Ferner kommen auch Fettsäureester von Polyoxyethylensorbitan wie das Polyoxyethylensorbitan-trioleat in Betracht.

Bei den kationischen Tensiden handelt es sich vor allem um quartäre Ammoniumsalze, welche als N-Substituenten mindestens einen Alkylrest mit 8 bis 22 C-Atomen enthalten und als weitere Substituenten niedrige, gegebenenfalls halogenierte Alkyl-, Benzyl- oder niedrige Hydroxyalkylreste aufweisen. Die Salze liegen vorzugsweise als Halogenide, Methylsulfate oder Ethylsulfate vor, z.B. das Stearyltrimethylammoniumchlorid oder das Benzyldi(2-chlorethyl)ethylammoniumbromid.

Die in der Formulierungstechnik gebräuchlichen Tenside sind u.a. in folgenden Publikationen beschrieben:

«Mc Cutcheon's Detergents and Emulsifiers Annual» MC Publishing Corpl, Ridgewood New Jersey, 1981;

Helmut Stache «Tensid-Taschenbuch» Carl Hanser-Verlag München/Wien 1981.

Die agrochemischen Zubereitungen enthalten in der Regel 0,1 bis 99%, insbesondere 0,1 bis 95% Wirkstoff der Formel I, 99,9 bis 1%, insbesondere 99,8 bis 5% eines festen oder flüssigen Zusatzstoffes darunter 0 bis 25%, insbesondere 0,1 bis 25% eines Tensides.

Während als Handelsware eher konzentrierte Mittel bevorzugt werden, verwendet der Endverbraucher in der Regel verdünnte Mittel.

Die Mittel können auch weitere Zusätze wie Stabilisatoren, Entschäumer, Viskositätsregulatoren, Bindemittel, Haftmittel sowie Dünger oder andere Wirkstoffe zur Erzielung spezieller Effekte enthalten.

Derartige agrochemische Mittel sind ein Bestandteil der vorliegenden Erfindung.

Die nachfolgenden Beispiele dienen zur näheren Erläuterung der Erfindung, ohne dieselbe zu beschränken. Temperaturen sind in Celsiusgraden angegeben. Prozente und Teile beziehen sich auf das Gewicht. RT bedeutet Raumtemperatur, h steht für Stunde, min für Minute, DMSO für Dimethylsulfoxid, THF für Tetrahydrofuran, DMF für Dimethylformamid.

Beispiel H1: Herstellung von

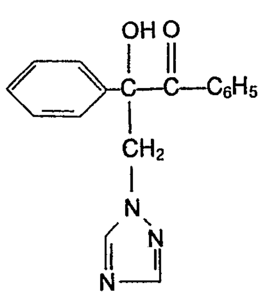

(1.1)

1-Benzoyl-1-Phenyl-2-[1H-1,2,4-triazol-1'-yl]-ethanol

Zur vorgelegten Lösung von 11,5 g Desylchlorid in 75 ml absolutem DMSO wurden bei 15°–30 °C unter Stickstoffatmosphäre Kühlen und gutem Rühren nacheinander 5 g Paraformaldehyd und 2,25 g 55%ige Natriumhydrid-Öldispersion in Portionen eingetragen, wobei gegen Schluss klare gelbe Lösung entstand und etwas Gasabgang zu beobachten war. Nach Abklingen der exothermen Reaktion wurde während 3 h gerührt und dann das Gemisch auf Raumtemperatur abgekühlt. Anschliessend wurde eine Lösung von 6,9 g 1,2,4-Triazol und 4,6 g Na-Salz von 1,2,4-Triazol in 75 ml absolutem DMSO zugetropft, wobei die Temperatur ohne Kühlen konstant blieb. Nach Rühren über Nacht bei Raumtemperatur wurde noch während 5 h auf 60° erwärmt, anschliessend abgekühlt und mit Eiswasser versetzt. Die trübe Lösung wurde mit verdünnter Salzsäure neutralisiert und mit Essigsäureethylester extrahiert. Nach dem Waschen mit Wasser, Trocknen und Eindampfen erhielt man ein Öl, welches nach Zugabe von etwas Diethylether kristallisierte. Filtration und Waschen mit Ether liefert bei 185–190 °C schmelzende Kristalle.

Beispiel H2: a) Herstellung des Ausgangsprodukts

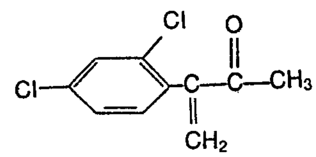

2-(2,4-Dichlorphenyl)-1-buten-2-on

30,5 g 2,4-Dichlorphenyl-aceton und 15,4 g N,N-Dimethylmethylenimmoniumchlorid wurden während 44 h unter Rückfluss gekocht. Anschliessend wurde auf Raumtemperatur abgekühlt, auf Eiswasser gegossen, mit Dichlormethan gewaschen und mit Essigsäureäthylester extrahiert. Der Essigester-Extrakt wurde partiell eingedampft und über eine mit Kieselgel gepackte Kurz-Säule (Methylenchlorid/Ether 1 : 1) filtriert, wobei nach Eindampfen das Produkt als braunrotes Öl erhalten wurde.

b) Herstellung eines weiteren Zwischenprodukts

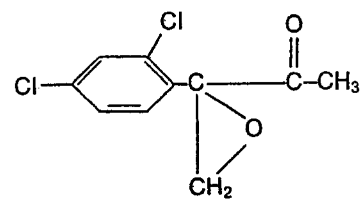

1-Acetyl-1-(2,4-Dichlorphenyl)-oxiran

Zu 13,3 g 2-(2,4-Dichlorphenyl)-1-buten-2-on in 225 ml Methanol wurden unter Rühren 22,2 ml Wasserstoffperoxid 30%ig und dann innert 0,75 h bei max. +3 °C unter Kühlen 12,4 ml 3N Natronlauge zugetropft. Nach weiterem Rühren während 5 h wurde mit Eiswasser versetzt, mit Methylenchlorid extrahiert, die Extrakte mit Wasser und

Natriumhydrogensulfit gewaschen (Peroxid-Test) und im Vakuum eingedampft. Nach dem Trocknen verblieben 7,3 g rohes Epoxid, das ohne weitere Reinigung eingesetzt werden konnte.

c) Herstellung des Endprodukts

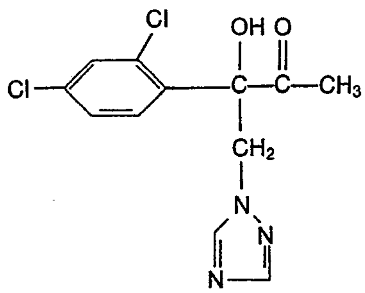

(1.2)

3-Hydroxy-3-(2,4-dichlorphenyl)-4-[1H-1,2,4-triazol-1'-yl]-2-butanon

Zur vorgelegten Lösung von 8,5 g freiem 1,2,4-Triazol sowie 5,6 g von dessen Natriumsalz in 80 ml absolutem DMSO wurden bei Raumtemperatur 7,3 g 1-Acetyl-1-(2,4-dichlorphenyl)-oxiran in 20 ml absolutem DMSO zugetropft und anschliessend während 6 h auf 40 °C erwärmt. Dann wurde auf Raumtemperatur abgekühlt, mit 1N Salzsäure neutralisiert, auf Eiswasser gegossen, der sich bildende Niederschlag abfiltriert und mit Wasser gut gewaschen. Nach Digerieren mit Ether-Hexan erhielt man das reine Produkt. Smp. 176–179 °C.

Auf analoge Weise lassen sich auch die nachfolgend aufgeführten Zwischen- und Endprodukte (sofern nicht speziell genannt, in Form von Diastereomerengemischen) herstellen:

Tabelle 1: Verbindungen der Formel

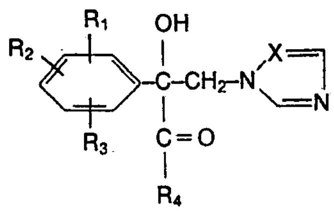

| Verb. Nr. | $R_1$ | $R_2$ | $R_3$ | $R_4$ | X | Physik. Konstante |
|---|---|---|---|---|---|---|
| 1.1 | H | H | H | $C_6H_5$ | N | Smp. 185–190° |
| 1.2 | 2–Cl | 4–Cl | H | $CH_3$ | N | Smp. 176–179° |
| 1.3 | H | H | H | $C(CH_3)_3$ | N | |
| 1.4 | H | H | H | $C_6H_3Cl_2(2,4)$ | N | |
| 1.5 | H | H | H | $C_6H_4Cl(4)$ | N | |
| 1.6 | 2–Cl | 4–Cl | H | $C_2H_5$ | N | Harz |
| 1.7 | 2–Cl | 4–Cl | H | $C_3H_7$–n | N | |
| 1.8 | 2–Cl | 4–Cl | H | $C_3H_7$–i | N | |
| 1.9 | 2–Cl | 4–Cl | H | $C_4H_9$–n | N | |
| 1.10 | 2–Cl | 4–Cl | H | $C_4H_9$–i | N | |
| 1.11 | 2–Cl | 4–Cl | H | $C_4H_9$–t | N | |
| 1.12 | 2–Cl | 4–Cl | H | $C_5H_{11}$–n | CH | |
| 1.13 | 2–Cl | 4–Cl | H | $C_6H_{13}$–n | N | |
| 1.14 | 2–Cl | 4–Cl | H | $C_8H_{17}$–n | N | |
| 1.15 | 2–Cl | 4–Cl | H | $C_{12}H_{25}$–n | N | |
| 1.16 | 2–Cl | 4–F | H | $CH_3$ | N | Harz |
| 1.17 | 2–Cl | 4–F | H | $C_2H_5$ | N | |
| 1.18 | 2–Cl | 4–F | H | $CH_3$ | CH | |
| 1.19 | 2–Cl | 4–F | H | $C_3H_7$–n | N | |
| 1.20 | 2–Cl | 4–F | H | $C_4H_9$–t | N | |
| 1.21 | 2–Cl | 4–F | H | $C_{12}H_{25}$–n | CH | |
| 1.22 | 2–Cl | 4–Br | H | $CH_3$ | N | |
| 1.23 | 2–Cl | 4–Br | H | $C_2H_5$ | N | |
| 1.24 | 2–Cl | 4–Br | H | $C_3H_7$–i | N | |
| 1.25 | 2–Cl | 4–Br | H | $C_4H_9$–n | N | |
| 1.26 | 2–Br | 4–Br | H | $CH_3$ | N | |
| 1.27 | H | 4–Cl | H | $C_2H_5$ | N | |
| 1.28 | H | 4–Br | H | $CH_3$ | N | |
| 1.29 | H | 4–F | H | $CH_3$ | CH | |
| 1.30 | 2–Cl | H | H | $CH_3$ | N | |
| 1.31 | 2–F | H | H | $C_2H_5$ | N | |
| 1.32 | 2–$CF_3$ | H | H | $CH_3$ | N | |
| 1.33 | H | 4–$CF_3$ | H | $C(CH_3)_3$ | N | |

Tabelle 1: (Fortsetzung)

| Verb. Nr. | $R_1$ | $R_2$ | $R_3$ | $R_4$ | X | Physik. Konstante |
|---|---|---|---|---|---|---|
| 1.34 | H | 4–$C_6H_5$ | H | $CH_3$ | N | |
| 1.35 | H | 4–$OC_6H_5$ | H | $CH_3$ | N | |
| 1.36 | H | 4–$OC_6H_4Cl(4)$ | H | $CH_3$ | N | Harz |
| 1.37 | H | 4–$OC_6H_4CH_3(4)$ | H | $CH_3$ | N | |
| 1.38 | 2–$CH_3$ | 4–$OC_6H_5$ | H | $CH_3$ | N | |
| 1.39 | 2–Cl | 4–Cl | H | Cyclopropyl | N | |
| 1.40 | 2–Cl | 4–Cl | H | Cyclobutyl | N | |
| 1.41 | 2–Cl | 4–Cl | H | Cyclopentyl | N | |
| 1.42 | 2–Cl | 4–Cl | H | Cyclohexyl | N | |
| 1.43 | 2–Cl | 4–F | H | Cyclopropyl | N | |
| 1.44 | 2–Cl | 4–Br | H | Cyclopropyl | N | |
| 1.45 | 2–Br | 4–Br | H | Cyclopentyl | N | |
| 1.46 | 2–Cl | 4–Cl | H | $C_6H_5$ | N | Smp. 98–99° |
| 1.47 | 2–Cl | 4–Cl | H | $C_6H_3Cl_2(2,4)$ | N | Smp. 167–168° |
| 1.48 | 2–Cl | 4–Cl | H | $C_6H_4F(4)$ | N | |
| 1.49 | 2–Cl | 4–Cl | 6–Cl | $C_6H_4Cl(2)$ | CH | |
| 1.50 | 2–Cl | 4–Cl | H | $C_6H_4F(2)$ | N | |
| 1.51 | H | 4–Cl | H | $C_2H_4Cl(4)$ | CH | |
| 1.52 | H | 4–Br | H | $C_6H_4Br(4)$ | N | |
| 1.53 | H | 4–$CF_3$ | H | $C_6H_4CF_3(4)$ | N | |
| 1.54 | 2–Cl | 4–Cl | H | H | N | Harz |
| 1.55 | 2–Cl | 4–F | H | H | N | |
| 1.56 | 2–$CH_3$ | H | H | H | N | |
| 1.57 | 3–$CF_3$ | H | H | H | N | |
| 1.58 | 2–Cl | 4–Cl | H | H | CH | |
| 1.59 | 2–Cl | 4–Cl | H | Cyclohexyl | CH | |
| 1.60 | 2–Cl | 4–Cl | H | $CH_3$ | CH | Smp. 203–204° |
| 1.61 | 2–Cl | 4–Cl | H | $C_6H_3(CH_3)_2(2,4)$ | N | Smp. 146–147° |
| 1.62 | 2–Cl | 4–Cl | H | $C_6H_5$ | CH | Smp. 234–235° |
| 1.63 | 2–Cl | 4–Cl | H | $C_6H_3Cl_2(2,4)$ | CH | Smp. 140–144° |
| 1.64 | 2–Cl | 4–Cl | H | $C_6H_3Cl_2(2,4)$ | CH | Öl |
| 1.65 | 3–$CF_3$ | H | H | $CH_3$ | N | |
| 1.66 | 3–$CF_3$ | H | H | $CH_3$ | CH | |
| 1.67 | 4–$OCH_3$ | H | H | $CH_3$ | N | |
| 1.68 | 2–$CH_3$ | 4–$OC_6H_4Cl(4)$ | H | $CH_3$ | N | |
| 1.69 | 2–$CH_3$ | 4–$OC_6H_4Br(4)$ | H | $C_2H_5$ | N | |
| 1.70 | 2–$CH_3$ | 4–$OC_6H_4Cl(4)$ | H | $CH_3$ | CH | |

Tabelle 2: Verbindungen der Formel

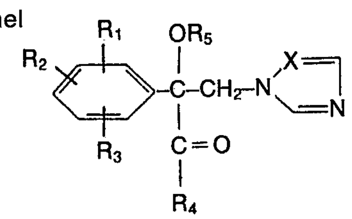

$R_5 = CH_3 = (1)$
$R_5 = C_2H_5 = (2)$
$R_5 = C_3H_7\text{–n} = (3)$

| Verb. Nr. | $R_1$ | $R_2$ | $R_3$ | $R_4$ | $R_5$ | X | Physik. Konstante |
|---|---|---|---|---|---|---|---|
| 2.1 | H | H | H | $C_6H_5$ | 1 | N | |
| 2.2 | 2–Cl | 4–Cl | H | $CH_3$ | 1 | N | Harz |
| 2.3 | H | H | H | $C(CH_3)_3$ | 1 | N | |
| 2.4 | H | H | H | $C_6H_3Cl_2(2,4)$ | 1 | N | |
| 2.5 | H | H | H | $C_6H_4Cl(4)$ | 1 | N | |
| 2.6 | 2–Cl | 4–Cl | H | $C_2H_5$ | 1 | N | |
| 2.7 | 2–Cl | 4–Cl | H | $C_3H_7$–n | 1 | N | |
| 2.8 | 2–Cl | 4–Cl | H | $C_3H_7$–i | 1 | N | |
| 2.9 | 2–Cl | 4–Cl | H | $C_4H_9$–n | 1 | N | |

Tabelle 2: (Fortsetzung)

| Verb. Nr. | $R_1$ | $R_2$ | $R_3$ | $R_4$ | $R_5$ | X | Physik. Konstante |
|---|---|---|---|---|---|---|---|
| 2.10 | 2–Cl | 4–Cl | H | $C_4H_9$–i | 3 | N | |
| 2.11 | 2–Cl | 4–Cl | H | $C_4H_9$–t | 1 | N | |
| 2.12 | 2–Cl | 4–Cl | H | $C_5H_{11}$–n | 1 | CH | |
| 2.13 | 2–Cl | 4–Cl | H | $C_6H_{13}$–n | 1 | N | |
| 2.14 | 2–Cl | 4–Cl | H | $C_8H_{17}$–n | 1 | N | |
| 2.15 | 2–Cl | 4–Cl | H | $C_{12}H_{25}$–n | 3 | N | |
| 2.16 | 2–Cl | 4–F | H | $CH_3$ | 1 | N | |
| 2.17 | 2–Cl | 4–F | H | $C_2H_5$ | 1 | N | |
| 2.18 | 2–Cl | 4–F | H | $CH_3$ | 1 | CH | |
| 2.19 | 2–Cl | 4–F | H | $C_3H_7$–n | 1 | N | |
| 2.20 | 2–Cl | 4–F | H | $C_4H_9$–t | 1 | N | |
| 2.21 | 2–Cl | 4–F | H | $C_{12}H_{25}$–n | 1 | CH | |
| 2.22 | 2–Cl | 4–Br | H | $CH_3$ | 1 | N | |
| 2.23 | 2–Cl | 4–Br | H | $C_2H_5$ | 2 | N | |
| 2.24 | 2–Cl | 4–Br | H | $C_3H_7$–i | 1 | N | |
| 2.25 | 2–Cl | 4–Br | H | $C_4H_9$–n | 1 | N | |
| 2.26 | 2–Br | 4–Br | H | $CH_3$ | 3 | N | |
| 2.27 | H | 4–Cl | H | $C_2H_5$ | 1 | N | |
| 2.28 | H | 4–Br | H | $CH_3$ | 1 | N | |
| 2.29 | H | 4–F | H | $CH_3$ | 1 | CH | |
| 2.30 | 2–Cl | H | H | $CH_3$ | 1 | N | |
| 2.31 | 2–F | H | H | $C_2H_5$ | 1 | N | |
| 2.32 | 2–$CF_3$ | H | H | $CH_3$ | 1 | N | |
| 2.33 | H | 4–$CF_3$ | H | $C(CH_3)_3$ | 1 | N | |
| 2.34 | H | 4–$C_6H_5$ | H | $CH_3$ | 1 | N | |
| 2.35 | H | 4–$OC_6H_5$ | H | $CH_3$ | 1 | N | |
| 2.36 | 2–$CH_3$ | 4–$OC_6H_4Cl$(4) | H | $CH_3$ | 1 | N | |
| 2.37 | H | 4–$OC_6H_4CH_3$(4) | H | $CH_3$ | 1 | N | |
| 2.38 | 2–$CH_3$ | 4–$OC_6H_4Br$(4) | H | $CH_3$ | 2 | N | |
| 2.39 | 2–Cl | 4–Cl | H | Cyclopropyl | 1 | N | |
| 2.40 | 2–Cl | 4–Cl | H | Cyclobutyl | 2 | N | |
| 2.41 | 2–Cl | 4–Cl | H | Cyclopentyl | 1 | N | |
| 2.42 | 2–Cl | 4–Cl | H | Cyclohexyl | 1 | N | |
| 2.43 | 2–Cl | 4–F | H | Cyclopropyl | 1 | N | |
| 2.44 | 2–Cl | 4–Br | H | Cyclopropyl | 1 | N | |
| 2.45 | 2–Br | 4–Br | H | Cyclopentyl | 1 | N | |
| 2.46 | 2–Cl | 4–Cl | H | $C_6H_5$ | 1 | N | |
| 2.47 | 2–Cl | 4–Cl | H | $C_6H_3Cl_2$(2,4) | 1 | N | |
| 2.48 | 2–Cl | 4–Cl | H | $C_6H_4F$(4) | 1 | N | |
| 2.49 | 2–Cl | 4–Cl | 6–Cl | $C_6H_4Cl$(2) | 1 | CH | |
| 2.50 | 2–Cl | 4–Cl | H | $C_6H_4F$(2) | 1 | N | |
| 2.51 | H | 4–Cl | H | $C_6H_4Cl$(4) | 1 | CH | |
| 2.52 | H | 4–Br | H | $C_6H_4Br$(4) | 1 | N | |
| 2.53 | H | 4–$CF_3$ | H | $C_6H_4CF_3$(4) | 1 | N | |
| 2.54 | 2–Cl | 4–Cl | H | H | 1 | N | Harz |
| 2.55 | 2–Cl | 4–F | H | H | 1 | N | |
| 2.56 | 2–$CH_3$ | H | H | H | 1 | N | |
| 2.57 | 3–$CF_3$ | H | H | H | 1 | N | |
| 2.58 | 2–Cl | 4–Cl | H | H | 1 | CH | |
| 2.59 | 2–Cl | 4–Cl | H | Cyclohexyl | 1 | CH | |
| 2.60 | 2–Cl | 4–Cl | H | $CH_3$ | 1 | CH | Smp. 203–204° |
| 2.61 | 2–Cl | 4–Cl | H | $C_6H_3(CH_3)_2$(2,4) | 1 | N | |
| 2.62 | 2–Cl | 4–Cl | H | $C_6H_3(CH_3)_2$(2,4) | 1 | N | |
| 2.63 | H | H | H | $CH_3$ | 1 | N | |
| 2.64 | 3–$CF_3$ | H | H | $CH_3$ | 1 | N | |

Tabelle 3: Verbindungen der Formel

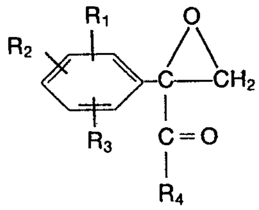

| Verb. Nr. | $R_1$ | $R_2$ | $R_3$ | $R_4$ |
|---|---|---|---|---|
| 3.1 | H | H | H | $C_6H_5$ (bekannt) |
| 3.2 | 2–Cl | 4–Cl | H | $CH_3$ |
| 3.3 | H | H | H | $C(CH_3)_3$ |
| 3.4 | H | H | H | $C_6H_3Cl_2(2,4)$ |
| 3.5 | H | H | H | $C_6H_4Cl(4)$ |
| 3.6 | 2–Cl | 4–Cl | H | $C_2H_5$ |
| 3.7 | 2–Cl | 4–Cl | H | $C_3H_7$–n |
| 3.8 | 2–Cl | 4–Cl | H | $C_3H_7$–i |
| 3.9 | 2–Cl | 4–Cl | H | $C_4H_9$–n |
| 3.10 | 2–Cl | 4–Cl | H | $C_4H_9$–i |
| 3.11 | 2–Cl | 4–Cl | H | $C_4H_9$–t |
| 3.12 | 2–Cl | 4–Cl | H | $C_5H_{11}$–n |
| 3.13 | 2–Cl | 4–Cl | H | $C_6H_{13}$–n |
| 3.14 | 2–Cl | 4–Cl | H | $C_8H_{17}$–n |
| 3.15 | 2–Cl | 4–Cl | H | $C_{12}H_{25}$–n |
| 3.16 | 2–Cl | 4–F | H | $CH_3$ |
| 3.17 | 2–Cl | 4–F | H | $C_2H_5$ |
| 3.18 | 2–Cl | 4–F | H | $CH_3$ |
| 3.19 | 2–Cl | 4–F | H | $C_3H_7$–n |
| 3.20 | 2–Cl | 4–F | H | $C_4H_9$–t |
| 3.21 | 2–Cl | 4–F | H | $C_{12}H_{25}$–n |
| 3.22 | 2–Cl | 4–Br | H | $CH_3$ |
| 3.23 | 2–Cl | 4–Br | H | $C_2H_5$ |
| 3.24 | 2–Cl | 4–Br | H | $C_3H_7$–i |
| 3.25 | 2–Cl | 4–Br | H | $C_4H_9$–n |
| 3.26 | 2–Br | 4–Br | H | $CH_3$ |
| 3.27 | H | 4–Cl | H | $C_2H_5$ |
| 3.28 | H | 4–Br | H | $CH_3$ |
| 3.29 | H | 4–F | H | $CH_3$ |
| 3.30 | 2–Cl | H | H | $CH_3$ |
| 3.31 | 2–F | H | H | $C_2H_5$ |
| 3.32 | 2–$CF_3$ | H | H | $CH_3$ |
| 3.33 | H | 4–$CF_3$ | H | $C(CH_3)_3$ |
| 3.34 | H | 4–$C_6H_5$ | H | $CH_3$ |
| 3.35 | H | 4–$OC_6H_5$ | H | $CH_3$ |
| 3.36 | H | 4–$OC_6H_4Cl(4)$ | H | $CH_3$ |
| 3.37 | H | 4–$OC_6H_4CH_3(4)$ | H | $CH_3$ |
| 3.38 | 2–$CH_3$ | 4–$OC_6H_5$ | H | $CH_3$ |
| 3.39 | 2–Cl | 4–Cl | H | Cyclopropyl |
| 3.40 | 2–Cl | 4–Cl | H | Cyclobutyl |
| 3.41 | 2–Cl | 4–Cl | H | Cyclopentyl |
| 3.42 | 2–Cl | 4–Cl | H | Cyclohexyl |
| 3.43 | 2–Cl | 4–F | H | Cyclopropyl |
| 3.44 | 2–Cl | 4–Br | H | Cyclopropyl |
| 3.45 | 2–Br | 4–Br | H | Cyclopentyl |
| 3.46 | 2–Cl | 4–Cl | H | $C_6H_5$ |
| 3.47 | 2–Cl | 4–Cl | H | $C_6H_3Cl_2(2,4)$ |
| 3.48 | 2–Cl | 4–Cl | H | $C_6H_4F(4)$ |
| 3.49 | 2–Cl | 4–Cl | 6–Cl | $C_6H_4Cl(2)$ |
| 3.50 | 2–Cl | 4–Cl | H | $C_6H_4F(2)$ |
| 3.51 | H | 4–Cl | H | $C_6H_4Cl(4)$ |
| 3.52 | H | 4–Br | H | $C_6H_4Br(4)$ |
| 3.53 | H | 4–$CF_3$ | H | $C_6H_4CF_3(4)$ |

Tabelle 3: (Fortsetzung)

| Verb. Nr. | $R_1$ | $R_2$ | $R_3$ | $R_4$ |
|---|---|---|---|---|
| 3.54 | 2–Cl | 4–Cl | H | H |
| 3.55 | 2–Cl | 4–F | H | H |
| 3.56 | 2–$CH_3$ | H | H | H |
| 3.57 | 3–$CF_3$ | H | H | H |
| 3.58 | 2–Cl | 4–Cl | H | H |
| 3.59 | 2–Cl | 4–Cl | H | Cyclohexyl |

Formulierungsbeispiele für flüssige Wirkstoffe der Formel I
(% = Gewichtsprozent)

| F1. Emulsions-Konzentrate | a) | b) | c) |
|---|---|---|---|
| Wirkstoff aus den Tabellen | 25% | 40% | 50% |
| Ca-Dodecylbenzolsulfonat | 5% | 8% | 6% |
| Ricinusöl-polyethylenglykolether (36 Mol Ethylenoxid) | 5% | – | – |
| Tributylphenoyl-polyethylenglykol-ether (30 Mol Ethylenoxid) | – | 12% | 4% |
| Cyclohexanon | – | 15% | 20% |
| Xylolgemisch | 65% | 25% | 20% |

Aus solchen Konzentraten können durch Verdünnen mit Wasser Emulsionen jeder gewünschten Konzentration hergestellt werden.

| F2. Lösungen | a) | b) | c) | d) |
|---|---|---|---|---|
| Wirkstoff aus den Tabellen | 80% | 10% | 5% | 95% |
| Ethylenglykol-monomethyl-ether | 20% | – | – | – |
| Polyethylenglykol MG 400 | – | 70% | – | – |
| N-Methyl-2-pyrrolidon | – | 20% | – | – |
| Epoxydiertes Kokosnussöl | – | – | 1% | 5% |
| Benzin (Siedegrenzen 160–190 °C) | – | – | 94% | – |

(MG = Molekulargewicht)

Die Lösungen sind zur Anwendung in Form kleinster Tropfen geeignet.

| F3. Granulate | a) | b) |
|---|---|---|
| Wirkstoff aus den Tabellen | 5% | 10% |
| Kaolin | 94% | – |
| Hochdisperse Kieselsäure | 1% | – |
| Attapulgit | – | 90% |

Der Wirkstoff wird in Methylenchlorid gelöst, auf den Träger aufgesprüht und das Lösungsmittel anschliessend im Vakuum abgedampft.

| F4. Stäubemittel | a) | b) |
|---|---|---|
| Wirkstoff aus den Tabellen | 2% | 5% |
| Hochdisperse Kieselsäure | 1% | 5% |
| Talkum | 97% | – |
| Kaolin | – | 90% |

Durch inniges Vermischen auf Trägerstoffe mit dem Wirkstoff enthält man gebrauchsfertige Stäubemittel.

Formulierungsbeispiele für feste Wirkstoffe der Formel I
(% = Gewichtsprozent)

| F5. Spritzpulver | a) | b) | c) |
|---|---|---|---|
| Wirkstoff aus den Tabellen | 25% | 50% | 75% |
| Na-Ligninsulfonat | 5% | 5% | – |
| Na-Laurylsulfat | 3% | – | 5% |
| Na-Diisobutylnaphthalinsulfonat | – | 6% | 10% |
| Octylphenolpolyethylenglykolether (7–8 Mol Ethylenoxid) | – | 2% | – |
| Hochdisperse Kieselsäure | 5% | 10% | 10% |
| Kaolin | 62% | 27% | – |

Der Wirkstoff wird mit den Zusatzstoffen gut vermischt und in einer geeigneten Mühle gut vermahlen. Man erhält Spritzpulver, die sich mit Wasser zu Suspensionen jeder gewünschten Konzentration verdünnen lassen.

F6. Emulsions-Konzentrat

| | |
|---|---|
| Wirkstoff aus den Tabellen | 10% |
| Octylphenolpolyethylenglykolether (4–5 Mol Ethylenoxid) | 3% |
| Ca-Dodecylbenzolsulfonat | 3% |
| Ricinusölpolyglykolether (35 Mol Ethylenoxid) | 4% |
| Cyclohexanon | 30% |
| Xylolgemisch | 30% |

Aus diesem Konzentrat können durch Verdünnen mit Wasser Emulsionen jeder gewünschten Konzentration hergestellt werden.

| F7. Stäubemittel | a) | b) |
|---|---|---|
| Wirkstoff aus den Tabellen | 5% | 8% |
| Talkum | 95% | – |
| Kaolin | – | 92% |

Man erhält anwendungsfertige Stäubemittel, indem der Wirkstoff mit den Träger vermischt und auf einer geeigneten Mühle vermahlen wird.

F8. Extruder-Granulat

| | |
|---|---|
| Wirkstoff aus den Tabellen | 10% |
| Na-Ligninsulfonat | 2% |
| Carboxymethylcellulose | 1% |
| Kaolin | 87% |

Der Wirkstoff wird mit den Zusatzstoffen vermischt, vermahlen und mit Wasser angefeuchtet. Dieses Gemisch wird extrudiert und anschliessend im Luftstrom getrocknet.

F9. Umhüllungs-Granulat

| | |
|---|---|
| Wirkstoff aus den Tabellen | 3% |
| Polyethylenglykol (MG 200) | 3% |
| Kaolin | 94% |

(MG = Molekulargewicht)

Der fein gemahlene Wirkstoff wird in einem Mischer auf das mit Polyethylenglykol angefeuchtete Kaolin gleichmässig aufgetragen. Auf diese Weise erhält man staubfreie Umhüllungs-Granulate.

F10. Suspensions-Konzentrat

| | |
|---|---|
| Wirkstoff aus den Tabellen | 40% |
| Ethylenglykol | 10% |
| Nonylphenolpolyethylenglykolether (15 Mol Ethylenoxid) | 6% |
| N-Ligninsulfonat | 10% |
| Carboxymethylcellulose | 1% |
| 37%ige wässrige Formaldehyd-Lösung | 0,2% |
| Silikonöl in Form einer 75%igen wässrigen Emulsion | 0,8% |
| Wasser | 32% |

Der fein gemahlene Wirkstoff wird mit den Zusatzstoffen innig vermischt. Man erhält so ein Suspensions-Konzentrat, aus welchem durch Verdünnen mit Wasser Suspensionen jeder gewünschten Konzentration hergestellt werden können.

Biologische Beispiele:

Beispiel B1: Wirkung gegen Puccinia graminis auf Weizen

a) Residual-protektive Wirkung

Weizenpflanzen wurden 6 Tage nach der Aussaat mit einer aus Spritzpulver des Wirkstoffes hergestellten Spritzbrühe (0,002% Aktivsubstanz) besprüht. Nach 24 Stunden wurden die behandelten Pflanzen mit einer Uredosporensuspension des Pilzes infiziert. Nach einer Inkubation während 48 Stunden bei 95–100% relativer Luftfeuchtigkeit und ca. 20 °C wurden die infizierten Pflanzen in einem Gewächshaus bei ca. 22 °C aufgestellt. Die Beurteilung der Rotpustelnentwicklung erfolgte 12 Tage nach der Infektion.

b) Systemische Wirkung

Zu Weizenpflanzen wurde 5 Tage nach der Aussaat eine aus Spritzpulver des Wirkstoffes hergestellte Spritzbrühe gegossen (0,006% Aktivsubstanz bezogen auf das Bodenvolumen). Nach 48 Stunden wurden die behandelten Pflanzen mit einer Ureidosporensuspension des Pilzes infiziert. Nach einer Inkubation während 48 Stunden bei 95–100% relativer Luftfeuchtigkeit und ca. 20 °C wurden die infizierten Pflanzen in einem Gewächshaus bei ca. 22 °C aufgestellt. Die Beurteilung der Rostpustelnentwicklung erfolgte 12 Tage nach der Infektion.

Verbindungen aus den Tabellen 1 und 2 zeigten gegen Puccinia-Pilze eine sehr gute Wirkung. Unbehandelte aber infizierte Kontrollpflanzen zeigten einen Puccinia-Befall von 100%. Unter anderem hemmten die Verbindungen 1.1, 1.2, 1.6, 1.16, 1.36, 1.46, 1.47, 1.54, 1.60–1.64 und 2.2 den Puccinia-Befall auf 0 bis 5%.

Beispiel B2: Wirkung gegen Cercospora arachidicola auf Erdnusspflanzen

Residual-protektive Wirkung

10–15 cm hohe Erdnusspflanzen wurden mit einer aus Spritzpulver der Wirksubstanz hergestellten Spritzbrühe (0,006% Aktivsubstanz) besprüht und 48 Stunden später mit einer Konidiensuspension des Pilzes infiziert. Die infizierten Pflanzen wurden während 72 Stunden bei ca. 21 °C und hoher Luftfeuchtigkeit inkubiert und anschliessend bis zum Auftreten der typischen Blattflecken in einem Gewächshaus aufgestellt. Die Beurteilung der fungiziden Wirkung erfolgte 12 Tage nach der Infektion basierend auf Anzahl und Grösse der auftretenden Flecken.

Im Vergleich zu unbehandelten, aber infizierten Kontrollpflanzen (Anzahl und Grösse der Flecken = 100%), zeigten Erdnusspflanzen, die mit Wirkstoffen aus den Tabellen 1 und 2 behandelt wurden, einen stark reduzierten Cercospora-Befall. So verhinderten die Verbindungen 1.1, 1.2, 1.6, 1.16, 1.36, 1.54 und 2.2 in obigen Versuchen das Auftreten von Flecken fast vollständig (0–10%).

Beispiel B3: Wirkung gegen Erysiphae graminis auf Gerste

a) Residual-protektive Wirkung

Ca. 8 cm hohe Gerstenpflanzen wurden mit einer aus Spritzpulver des Wirkstoffes hergestellten Spritzbrühe (0,002% Aktivsubstanz) besprüht. Nach 3–4 Stunden wurden die behandelten Pflanzen mit Konidien des Pilzes bestäubt. Die infizierten Gerstenpflanzen wurden in einem Gewächshaus bei ca. 22 °C aufgestellt und der Pilzbefall nach 10 Tagen beurteilt.

b) Systemische Wirkung

Zu ca. 8 cm hohen Gerstenpflanzen wurde eine aus Spritzpulver des Wirkstoffes hergestellte Spritzbrühe gegossen (0,002% Aktivsubstanz bezogen auf das Erdvolumen). Es wurde dabei darauf geachtet, dass die Spritzbrühe nicht mit den oberirdischen Pflanzenteilen in Berührung kam. Nach 48 Stunden wurden die behandelten Pflanzen mit Konidien des Pilzes bestäubt. Die infizierten Gerstenpflanzen wurden in einem Gewächshaus bei ca. 22 °C aufgestellt und der Pilzbefall nach 10 Tagen beurteilt.

Verbindungen der Formel I zeigten eine gute Wirkung gegen Erysiphe-Pilze. Unbehandelte, aber infizierte Kontrollpflanzen zeigten einen Erysiphe-Befall von 100%. Unter anderen Verbindungen aus den Tabellen 1 und 2 hemmten die Verbindungen Nr. 1.1, 1.2, 1.6, 1.16, 1.54 und 2.2 den Pilzbefall bei Gerste auf 0 bis 5%.

Beispiel B4: Residual-protektive Wirkung gegen Venturia inaequalis auf Apfeltrieben

Apfelstecklinge mit 10–20 cm langen Frischtrieben wurden mit einer aus Spritzpulver des Wirkstoffes hergestellten Spritzbrühe (0,006% Aktivsubstanz) besprüht. Nach 24 Stunden wurden die behandelten Pflanzen mit einer Konidiensuspension des Pilzes infiziert. Die Pflanzen wurden dann während 5 Tagen bei 90–100% relativer Luftfeuchtigkeit inkubiert und während 10 weiteren Tagen in einem Gewächshaus bei 20–24 °C aufgestellt. Der Schorfbefall wurde 15 Tage nach der Infektion beurteilt. Verbindungen 1.1, 1.2, 1.6, 1.47, 1.60 bis 1.64 und 2.2 und andere hemmten den Krankheitsbefall auf weniger als 20%. Unbehandelte aber infizierte Triebe zeigten einen 100%igen Venturia-Befall.

Beispiel B5: Wirkung gegen Botrytis cinerea auf Äpfeln

Residual-protektive Wirkung

Künstlich verletzte Äpfel wurden behandelt, indem eine aus Spritzpulver der Wirksubstanz hergestellte Spritzbrühe (0,02% Aktivsubstanz) auf die Verletzungsstellen aufgetropft wurde. Die behandelten Früchte wurden anschliessend mit einer Sporensuspension von Botrytis cinerea inokuliert und während einer Woche bei hoher Luftfeuchtigkeit und ca. 20 °C inkubiert.

Das Vorhandensein und die Grösse der Fäulnis-Stellen an der Frucht dienten zur Bewertung der fungiziden Aktivität. Bei Behandlung mit Verbindungen aus den Tabellen 1 und 2, z. B. Nr. 1.1, 1.6, 1.46 und 2.2, wurden keine oder fast keine Fäulnis-Stellen (0–5% Befall) beobachtet.

**Patentansprüche für die Vertragsstaaten: BE, CH, DE, FR, GB, IT, LI, LU, NL, SE**

1. Verbindungen der Formel I

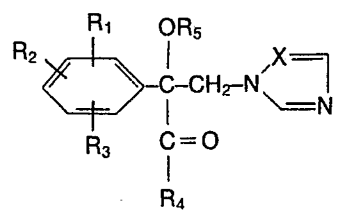

(I)

worin

$R_1$, $R_2$ und $R_3$ unabhängig voneinander für Wasserstoff, Halogen, $C_1$–$C_4$-Alkyl, $CF_3$, Phenyl oder durch $C_1$–$C_4$-Alkyl, $C_1$–$C_4$-Alkoxy, $C_1$–$C_3$-Haloalkyl, Halogen und/oder Cyano substituiertes Phenyl oder Phenoxy oder durch $C_1$–$C_4$-Alkyl, $C_1$–$C_4$-Alkoxy, $C_1$–$C_3$-Haloalkyl, Halogen und/oder Cyano substituiertes Phenoxy stehen;

$R_4$ Wasserstoff, $C_1$–$C_{10}$-Alkyl, $C_3$–$C_6$-Cycloalkyl, Phenyl oder durch $C_1$–$C_4$-Alkyl, $C_1$–$C_4$-Alkoxy, $C_1$–$C_3$-Haloalkyl, Halogen und/oder Cyano substituiertes Phenyl bedeutet;

X für –CH= oder –N= steht; und

$R_5$ Wasserstoff, $C_1$–$C_{12}$-Alkyl, $C_2$–$C_4$-Alkenyl, $C_2$–$C_4$-Alkinyl, Benzyl oder durch $C_1$–$C_4$-Alkyl, $C_1$–$C_4$-Alkoxy, $C_1$–$C_3$-Haloalkyl, Halogen und/oder Cyano substituiertes Benzyl bedeutet; unter Einschluss ihrer Säureadditionssalze, quaternären Azoliumsalze und Metallkomplexe.

2. Verbindungen der Formel I nach Anspruch 1, worin $R_1$, $R_2$ und $R_3$ unabhängig voneinander für Wasserstoff, Halogen, $C_1$–$C_3$-Alkyl, $CF_3$, Phenyl, Phenoxy oder durch $C_1$–$C_4$-Alkyl, $C_1$–$C_4$-Alkoxy, $C_1$–$C_3$-Haloalkyl, Halogen oder Cyano substituiertes Phenyl oder Phenoxy stehen;

$R_4$ Wasserstoff, $C_1$–$C_6$-Alkyl, $C_3$–$C_6$-Cycloalkyl, Phenyl oder durch $C_1$–$C_4$-Alkyl, $C_1$–$C_4$-Alkoxy, $C_1$–$C_3$-Haloalkyl, Halogen oder Cyano substituiertes Phenyl bedeutet; $R_5$ für Wasserstoff, $C_1$–$C_8$-Alkyl, $C_2$–$C_4$-Alkenyl, Propargyl, Benzyl oder ein- oder zweifach durch Fluor, Chlor, Brom und/oder $C_1$–$C_3$-Alkyl substituiertes Benzyl steht und X für –CH= oder –N= steht; unter Einschluss ihrer Säureadditionssalze, quaternären Azoliumsalze und Metallkomplexe.

3. Verbindungen der Formel I nach Anspruch 2, worin $R_1$, $R_2$ und $R_3$ unabhängig voneinander für Wasserstoff, Fluor, Chlor, Brom, Methyl, Ethyl, $CF_3$, Phenyl, Phenoxy, $C_6H_4Cl(4)$, $C_6H_4CH_3(4)$, $C_6H_3Cl_2(2,4)$, $OC_6H_4Cl(4)$, $OC_6H_4CH_3(4)$ oder $OC_6H_3Cl_2(2,4)$ steht;

$R_4$ Wasserstoff, $C_1$–$C_4$-Alkyl, $C_3$–$C_6$-Cycloalkyl, Phenyl oder durch Methyl, Methoxy, $CF_3$, F, Cl, Br oder CN substituiertes Phenyl bedeutet; $R_5$ für Wasserstoff, $C_1$–$C_6$-Alkyl, $C_3$–$C_4$-Alkenyl, Propargyl, Benzyl oder ein- oder zweifach durch Fluor, Chlor und/oder Methyl substituiertes Benzyl steht; und X für –N= steht; unter Einschluss ihrer Säureadditionssalze, quaternären Azoliumsalze und Metallkomplexe.

4. Verbindungen der Formel I nach Anspruch 3, worin $R_1$ für Wasserstoff, 2-Cl, 2-Br, 2F, 2-$CF_3$, 2-$C_6H_5$, 2-$OC_6H_5$, 3-F, 3–Cl, 3-Br, 3-$CF_3$, 3-$C_6H_5$ oder 3-$OC_6H_5$ steht; $R_2$ Wasserstoff, 4-Cl, 4-Br, 4-F, 4-$CF_3$, 4-$C_6H_5$, 4-$OC_6H_5$, 4-$C_6H_4Cl(4)$, 4-$C_6H_4CH_3(4)$, 4-$C_6H_3Cl_2(2,4)$, 4-$OC_6H_4Cl(4)$, 4-$OC_6H_4CH_3(4)$, 4-$OC_6H_3Cl_2(2,4)$, 4-O-$C_6H_4F(4)$, 5-Cl, 5-Br, 5-F oder 5-$CF_3$ bedeutet; $R_3$ für Wasserstoff, steht;

$R_4$ für Wasserstoff, Methyl, Phenyl oder 2,4-Dichlorphenyl steht; $R_5$ Wasserstoff, $C_1$–$C_5$-Alkyl, Allyl, Propargyl, Benzyl, 2-Halobenzyl, 4-Halobenzyl, 2,4-Dihalobenzyl, 2,6-Dihalobenzyl oder 3,4-Dihalobenzyl bedeutet und X für –N= steht; unter Einschluss der Säureadditionssalze, quaternären Azoliumsalze und Metallkomplexe.

5. Verbindungen der Formel I nach Anspruch 4, worin $R_1$ für 2-H, 2-F, 2-Cl oder 2-Br steht; $R_2$ 4-F, 4-Cl, 4-Br oder 4-$CF_3$ bedeutet; $R_3$ für Wasserstoff steht;

$R_4$ für Wasserstoff, Methyl, Phenyl oder 2,4-Dichlorphenyl steht; $R_5$ Wasserstoff, $C_1$–$C_5$-Alkyl, Allyl, Propargyl, Benzyl, 2,6-Dichlorbenzyl, 4-Chlorbenzyl, 4-Fluorbenzyl oder 2,4-Dichlorbenzyl bedeutet; und X für –N= steht; unter Einschluss ihrer Säureadditionssalze, quaternären Azoliumsalze und Metallkomplexe.

6. Eine Verbindung der Formel I nach Anspruch 1, ausgewählt aus der Reihe:

2-(2,4-Dichlorphenyl)-2-hydroxy-3-(1H-1,2,4-triazol-1'-yl)-propanal (Verb. Nr. 1.54);
2-(2,4-Dichlorphenyl)-2-hydroxy-1-(1H-1,2,4-triazol-1'-yl)-butan-3-on (Verb. Nr. 1.2);
2-(2,4-Dichlorphenyl)-2-hydroxy-1-(1H-1,2,4-triazol-1'-yl)-pentan-3-on (Verb. Nr. 1.6);
2-(2,4-Dichlorphenyl)-2-methoxy-1-(1H-1,2,4-triazol-1'-yl)-butan-3-on (Verb. Nr. 2.2);
2-(2-Chlor-4-fluorphenyl)-2-hydroxy-1-(1H-1,2,4-triazol-1'-yl)-butan-3-on (Verb. Nr. 1.16);
2-[2-Methyl-4-(4'-chlorphenoxy)]-2-hydroxy-1-(1H-1,2,4-triazol-1'-yl)-butan-3-on (Verb. Nr. 1.36).

7. Verfahren zur Herstellung der in Anspruch 1 definierten Verbindungen der Formel I, dadurch gekennzeichnet, dass man entweder ein Oxiran der Formel II

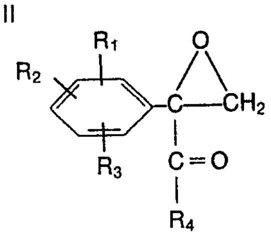

(II)

mit einem Azol der Formel III

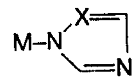

(III)

zuerst zu einer Verbindung der Formel Ia

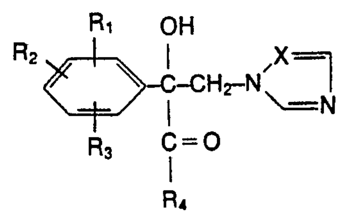

(Ia)

umsetzt, oder dadurch dass man ein α-Haloketon bzw. α-Haloaldehyd der Formel IV

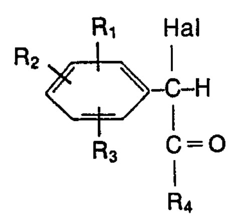

(IV)

mit Paraformaldehyd und einer starken nicht nukleophilen Base reagieren lässt und durch anschliessende Zugabe eines Azols der Formel III zu einem entsprechenden Alkohol der Formel Ia weiterreagieren lässt und in beiden Fällen, sofern gewünscht, dem Alkohol Ia auf übliche Weise, z.B. durch Reaktion mit einer Verbindung der Formel V

$$R_5\text{–}W \qquad (V)$$

in einen Ether der Formel I überführt, wobei die Substituenten $R_1$, $R_2$, $R_3$, $R_4$, $R_5$ und X in den Formeln Ia, II, III, IV und V die unter Formel I angegebenen Bedeutungen haben, M für Wasserstoff

oder ein Metallatom steht, Hal Halogen und W für OH oder eine übliche Abgangsgruppe steht.

8. Mittel zur Bekämpfung oder Verhütung eines Befalls durch Mikroorganismen und/oder zur Regulierung des Pflanzenwachstums, dadurch gekennzeichnet, dass es als mindestens eine aktive Komponente eine Verbindung der Formel I gemäss einem der Ansprüche 1 bis 6 zusammen mit üblichen Zuschlagstoffen enthält.

9. Verfahren zur Bekämpfung oder Verhütung eines Befalls von Kulturpflanzen durch phytopathogene Mikroorganismen und/oder zur Regulierung des Pflanzenwachstums, dadurch gekennzeichnet, dass man eine gemäss den Ansprüchen 1 bis 6 definierte Verbindung der Formel I auf die Pflanze oder deren Standort appliziert.

10. Verwendung von Verbindungen der Formel I gemäss Anspruch 1 zur Bekämpfung und/oder zur präventiven Verhütung eines Befalls von Mikroorganismen.

11. Die Oxirane der Formel II

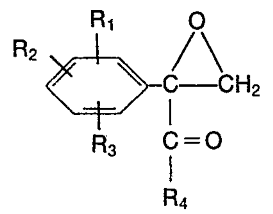

(II)

worin $R_1$ bis $R_4$ wie unter Formel I definiert sind, mit Ausnahme der Verbindungen 2-Formyl-2-phenyl-oxiran und 2-Benzoyl-2-phenyl-oxiran.

**Patentansprüche für den Vertragsstaat AT**

1. Mikrobizides Mittel, dadurch gekennzeichnet, dass es neben üblichen Zuschlagstoffen als aktive Komponente mindestens eine Verbindung der Formel I enthält,

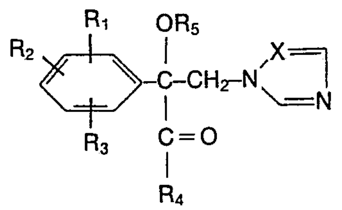

(I)

worin

$R_1$, $R_2$ und $R_3$ unabhängig voneinander für Wasserstoff, Halogen, $C_1$–$C_4$-Alkyl, $CF_3$, Phenyl oder durch $C_1$–$C_4$-Alkyl, $C_1$–$C_4$-Alkoxy, $C_1$–$C_3$-Haloalkyl, Halogen und/oder Cyano substituiertes Phenyl oder Phenoxy oder durch $C_1$–$C_4$-Alkyl, $C_1$–$C_4$-Alkoxy, $C_1$–$C_3$-Haloalkyl, Halogen und/oder Cyano substituiertes Phenoxy stehen;

$R_4$ Wasserstoff, $C_1$–$C_{10}$-Alkyl, $C_3$–$C_6$-Cycloalkyl, Phenyl oder durch $C_1$–$C_4$-Alkyl, $C_1$–$C_4$-Alkoxy, $C_1$–$C_3$-Haloalkyl, Halogen und/oder Cyano substituiertes Phenyl bedeutet;

X für –CH= oder –N= steht; und

$R_5$ Wasserstoff, $C_1$–$C_{12}$-Alkyl, $C_2$–$C_4$-Alkenyl, $C_2$–$C_4$-Alkinyl, Benzyl oder durch $C_1$–$C_4$-Alkyl, $C_1$–$C_4$-Alkoxy, $C_1$–$C_3$-Haloalkyl, Halogen und/oder Cyano substituiertes Benzyl bedeutet; unter Einschluss ihrer Säureadditionssalze, quaternären Azoliumsalze und Metallkomplexe.

2. Mittel nach Anspruch 1, enthaltend als aktive Komponente mindestens eine Verbindung der Formel I, worin $R_1$, $R_2$ und $R_3$ unabhängig voneinander für Wasserstoff, Halogen, $C_1$–$C_3$-Alkyl, $CF_3$, Phenyl, Phenoxy oder durch $C_1$–$C_4$-Alkyl, $C_1$–$C_4$-Alkoxy, $C_1$–$C_3$-Haloalkyl, Halogen oder Cyano substituiertes Phenyl oder Phenoxy stehen;

$R_4$ Wasserstoff, $C_1$–$C_6$-Alkyl, $C_3$–$C_6$-Cycloalkyl, Phenyl oder durch $C_1$–$C_4$-Alkyl, $C_1$–$C_4$-Alkoxy, $C_1$–$C_3$-Haloalkyl, Halogen oder Cyano substituiertes Phenyl bedeutet; $R_5$ für Wasserstoff, $C_1$–$C_8$-Alkyl, $C_2$–$C_4$-Alkenyl, Propargyl, Benzyl oder ein- oder zweifach durch Fluor, Chlor, Brom und/oder $C_1$–$C_3$-Alkyl substituiertes Benzyl steht und X für –CH= oder –N= steht; unter Einschluss ihrer Säureadditionssalze, quaternären Azoliumsalze und Metallkomplexe.

3. Mittel nach Anspruch 2, enthaltend als aktive Komponente mindestens eine Verbindung der Formel I, worin $R_1$, $R_2$ und $R_3$ unabhängig voneinander für Wasserstoff, Fluor, Chlor, Brom, Methyl, Ethyl, $CF_3$, Phenyl, Phenoxy, $C_6H_4Cl(4)$, $C_6H_4CH_3(4)$, $C_6H_3Cl_2(2,4)$, $OC_6H_4Cl(4)$, $OC_6H_4CH_3(4)$ oder $OC_6H_3Cl_2(2,4)$ steht;

$R_4$ Wasserstoff, $C_1$–$C_4$-Alkyl, $C_3$–$C_6$-Cycloalkyl, Phenyl oder durch Methyl, Methoxy, $CF_3$, F, Cl, Br oder CN substituiertes Phenyl bedeutet; $R_5$ für Wasserstoff, $C_1$–$C_6$-Alkyl, $C_3$–$C_4$-Alkenyl, Propargyl, Benzyl oder ein- oder zweifach durch Fluor, Chlor und/oder Methyl substituiertes Benzyl steht; und X für –N= steht; unter Einschluss ihrer Säureadditionssalze, quaternären Azoliumsalze und Metallkomplexe.

4. Mittel nach Anspruch 3, enthaltend als aktive Komponente mindestens eine Verbindung der Formel I, worin $R_1$ für Wasserstoff, 2-Cl, 2-Br, 2-F, 2-$CF_3$, 2-$C_6H_5$, 2-$OC_6H_5$, 3-F, 3-Cl, 3-Br, 3-$CF_3$, 3-$C_6H_5$ oder 3-$OC_6H_5$ steht; $R_2$ Wasserstoff, 4-Cl, 4-Br, 4-F, 4-$CF_3$, 4-$C_6H_5$, 4-$OC_6H_5$, 4-$C_6H_4Cl(4)$, 4-$C_6H_4CH_3(4)$, 4-$C_6H_3Cl_2(2,4)$, 4-$OC_6H_4Cl(4)$, 4-$OC_6H_4CH_3(4)$, 4-$OC_6H_3Cl_2(2,4)$, 4-O-$C_6H_4F(4)$, 5-Cl, 5-Br, 5-F oder 5-$CF_3$ bedeutet; $R_3$ für Wasserstoff, steht;

$R_4$ für Wasserstoff, Methyl, Phenyl oder 2,4-Dichlorphenyl steht; $R_5$ Wasserstoff, $C_1$–$C_5$-Alkyl, Allyl, Propargyl, Benzyl, 2-Halobenzyl, 4-Halobenzyl, 2,4-Dihalobenzyl, 2,6-Dihalobenzyl oder 3,4-Dihalobenzyl bedeutet und X für –N= steht; unter Einschluss der Säureadditionssalze, quaternären Azoliumsalze und Metallkomplexe.

5. Mittel nach Anspruch 4, enthaltend als aktive Komponente mindestens eine Verbindung der Formel I, worin $R_1$ für 2-H, 2-F, 2-Cl oder 2-Br steht; $R_2$ 4-F, 4-Cl, 4-Br oder 4-$CF_3$ bedeutet; $R_3$ für Wasserstoff steht;

$R_4$ für Wasserstoff, Methyl, Phenyl oder 2,4-Dichlorphenyl steht; $R_5$ Wasserstoff, $C_1$–$C_5$-Alkyl, Allyl, Propargyl, Benzyl, 2,6-Dichlorbenzyl, 4-Chlorbenzyl, 4-Fluorbenzyl oder 2,4-Dichlorbenzyl bedeutet; und X für –N= steht; unter Einschluss ihrer Säureadditionssalze, quaternären Azoliumsalze und Metallkomplexe.

6. Mittel nach Anspruch 1, enthaltend als aktive Komponente mindestens eine Verbindung der Formel I, ausgewählt aus der Reihe:

2-(2,4-Dichlorphenyl)-2-hydroxy-3-(1H-1,2,4-triazol-1′-yl)-propanal (Verb. Nr. 1.54);
2-(2,4-Dichlorphenyl)-2-hydroxy-1-(1H-1,2,4-triazol-1′-yl)-butan-3-on (Verb. Nr. 1.2);
2-(2,4-Dichlorphenyl)-2-hydroxy-1-(1H-1,2,4-triazol-1′-yl)-pentan-3-on (Verb. Nr. 1.6);
2-(2,4-Dichlorphenyl)-2-methoxy-1-(1H-1,2,4-triazol-1′-yl)-butan-3-on (Verb. Nr. 2.2);
2-(2-Chlor-4-fluorphenyl)-2-hydroxy-1-(1H-1,2,4-triazol-1′-yl)-butan-3-on (Verb. Nr. 1.16);
2-[2-Methyl-4-(4′-chlorphenoxy)]-2-hydroxy-1-(1H-1,2,4-triazol-1′-yl)-butan-3-on (Verb. Nr. 1.36).

7. Verfahren zur Herstellung der in Anspruch 1 definierten Verbindungen der Formel I, dadurch gekennzeichnet, dass man entweder ein Oxiran der Formel II

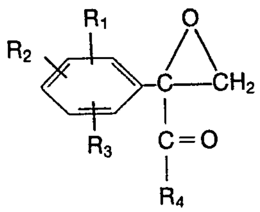

(II)

mit einem Azol der Formel III

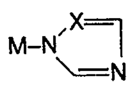

(III)

zuerst zu einer Verbindung der Formel Ia

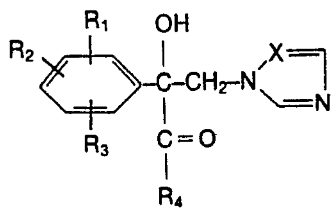

(Ia)

umsetzt, oder dadurch dass man ein α-Haloketon bzw. α-Haloaldehyd der Formel IV

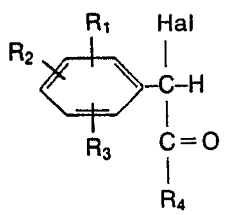

(IV)

mit Paraformaldehyd und einer starken nicht nukleophilen Base reagieren lässt und durch anschliessende Zugabe eines Azols der Formel III zu einem entsprechenden Alkohol der Formel Ia weiterreagieren lässt und in beiden Fällen, sofern gewünscht, dem Alkohol Ia auf übliche Weise, z.B. durch Reaktion mit einer Verbindung der Formel V

$$R_5\text{–}W \qquad (V)$$

in einen Ether der Formel I überführt, wobei die Substituenten $R_1$, $R_2$, $R_3$, $R_4$, $R_5$ und X in den Formeln Ia, II, III, IV und V die unter Formel I angegebenen Bedeutungen haben, M für Wasserstoff oder ein Metallatom steht, Hal Halogen und W für OH oder eine übliche Abgangsgruppe steht.

8. Verfahren zur Herstellung eines agrochemischen Mittels, dadurch gekennzeichnet, dass man mindestens eine gemäss einem der Ansprüche 1 bis 6 definierte Verbindung der Formel I mit geeigneten festen oder flüssigen Zusatzstoffen und Tensiden innig vermischt.

9. Verfahren zur Bekämpfung oder Verhütung eines Befalls von Kulturpflanzen durch phytopathogene Mikroorganismen und/oder zur Regulierung des Pflanzenwachstums, dadurch gekennzeichnet, dass man eine gemäss den Ansprüchen 1 bis 6 definierte Verbindung der Formel I auf die Pflanze oder deren Standort appliziert.

10. Verwendung von Verbindungen der Formel I gemäss Anspruch 1 zur Bekämpfung und/oder zur präventiven Verhütung eines Befalls von Mikroorganismen.

11. Verwendung von Oxiranen der Formel II

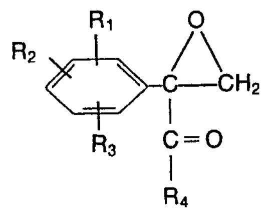

(II)

worin $R_1$ bis $R_4$ wie unter Formel I definiert sind, mit Ausnahme der Verbindungen 2-Formyl-2-phenyl-oxiran und 2-Benzoyl-2-phenyl-oxiran.

**Claims for the contracting states: BE, CH, DE, FR, GB, IT, LI, LU, NL, SE.**

1. Compounds of the formula I

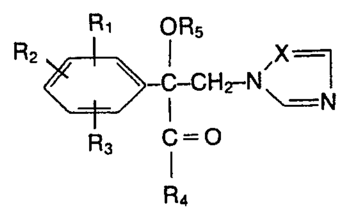

(I)

wherein

$R_1$, $R_2$ and $R_3$ are each independently hydrogen, halogen, $C_1$–$C_4$-alkyl, $CF_3$, phenyl, phenyl substituted by $C_1$–$C_4$-alkyl, $C_1$–$C_4$-alkoxy, $C_1$–$C_3$-haloalkyl, halogen and/or by cyano, phenoxy or phenoxy substituted by $C_1$–$C_4$-alkyl, $C_1$–$C_4$-alkoxy, $C_1$–$C_3$-haloalkyl, halogen and/or by cyano;

$R_4$ is hydrogen, $C_1$–$C_{10}$-alkyl, $C_3$–$C_6$-cycloalkyl, phenyl or phenyl substituted by $C_1$–$C_4$-alkyl, $C_1$–$C_4$-alkoxy, $C_1$–$C_3$-haloalkyl, halogen and/or by cyano;

X is –CH= or –N=; and

$R_5$ is hydrogen, $C_1$–$C_{12}$-alkyl, $C_2$–$C_4$-alkenyl, $C_2$–$C_4$-alkynyl, benzyl or benzyl substituted by $C_1$–$C_4$-alkyl, $C_1$–$C_4$-alkoxy, $C_1$–$C_3$-haloalkyl, halogen and/or by cyano; including the acid addition salts, quaternary azolium salts and metal complexes thereof.

2. Compounds of the formula I according to claim 1 wherein $R_1$, $R_2$ and $R_3$ are each independently hydrogen, halogen, $C_1$–$C_3$-alkyl, $CF_3$, phenyl, phenoxy or phenyl or phenoxy substituted by $C_1$–$C_4$-alkyl, $C_1$–$C_4$-alkoxy, $C_1$–$C_3$-haloalkyl, halogen or by cyano; $R_4$ is hydrogen, $C_1$–$C_6$-alkyl, $C_3$–$C_6$-cycloalkyl, phenyl or phenyl substituted by $C_1$–$C_4$-alkyl, $C_1$–$C_4$-alkoxy, $C_1$–$C_3$-haloalkyl, halogen or by cyano; $R_5$ is hydrogen, $C_1$–$C_8$-alkyl, $C_2$–$C_4$-alkenyl, propargyl, benzyl or benzyl mono- or di-substituted by fluorine, chlorine, bromine and/or by $C_1$–$C_3$-alkyl, and X is –CH= or –N=; including the acid addition salts, quaternary azolium salts and metal complexes thereof.

3. Compounds of the formula I according to claim 2 wherein $R_1$, $R_2$ and $R_3$ are each independently hydrogen, fluorine, chlorine, bromine, methyl, ethyl, $CF_3$, phenyl, phenoxy, $C_6H_4Cl(4)$, $C_6H_4CH_3(4)$, $C_6H_3Cl_2(2,4)$, $OC_6H_4Cl(4)$, $OC_6H_4CH_3(4)$ or $OC_6H_3Cl_2(2,4)$; $R_4$ is hydrogen, $C_1$–$C_4$-alkyl, $C_3$–$C_6$-cycloalkyl, phenyl, or phenyl substituted by methyl, methoxy, $CF_3$, F, Cl, Br or by CN; $R_5$ is hydrogen, $C_1$–$C_6$-alkyl, $C_3$–$C_4$-alkenyl, propargyl, benzyl or benzyl mono- or di-substituted by fluorine, chlorine and/or by methyl; and X is –N=; including the acid addition salts, quaternary azolium salts and metal complexes thereof.

4. Compounds of the formula I according to claim 3 wherein $R_1$ is hydrogen, 2-Cl, 2-Br, 2-F, 2-$CF_3$, 2-$C_6H_5$, 2-$OC_6H_5$, 3-F, 3-Cl, 3-Br, 3-$CF_3$, 3-$C_6H_5$ or 3-$OC_6H_5$; $R_2$ is hydrogen, 4-Cl, 4-Br, 4-F, 4-$CF_3$, 4-$C_6H_5$, 4-$OC_6H_5$, 4-$C_6H_4Cl(4)$, 4-$C_6H_4CH_3(4)$, 4-$C_6H_3Cl_2(2,4)$, 4-$OC_6H_4Cl(4)$, 4-$OC_6H_4CH_3(4)$, 4-$OC_6H_3Cl_2(2,4)$, 4-O-$C_6H_4F(4)$, 5-Cl, 5-Br, 5-F or 5-$CF_3$; $R_3$ is hydrogen; $R_4$ is hydrogen, methyl, phenyl or 2,4-dichlorophenyl; $R_5$ is hydrogen, $C_1$–$C_5$-alkyl, allyl, propargyl, benzyl, 2-halobenzyl, 4-halobenzyl, 2,4-dihalobenzyl, 2,6-dihalobenzyl or 3,4-dihalobenzyl and X is –N=; including the acid addition salts, quaternary azolium salts and metal complexes thereof.

5. Compounds of the formula I according to claim 4 wherein $R_1$ is 2-H, 2-F, 2-Cl or 2-Br; $R_2$ is 4-F, 4-Cl, 4-Br or 4-$CF_3$; $R_3$ is hydrogen; $R_4$ is hydrogen, methyl, phenyl or 2,4-dichlorophenyl; $R_5$ is hydrogen, $C_1$–$C_5$-alkyl, allyl, propargyl, benzyl, 2,6-dichlorobenzyl, 4-chlorobenzyl, 4-fluorobenzyl or 2,4-dichlorobenzyl; and X is –N=; including the acid addition salts, quaternary azolium salts and metal complexes thereof.

6. A compound of the formula I according to claim 1 selected from the group consisting of:
2-(2,4-dichlorophenyl)-2-hydroxy-3-(1H-1,2,4-triazol-1′-yl)-propanal (compound No. 1.54);
2-(2,4-dichlorophenyl)-2-hydroxy-1-(1H-1,2,4-triazol-1′-yl)-butan-3-one (compound No. 1.2);
2-(2,4-dichlorophenyl)-2-hydroxy-1-(1H-1,2,4-triazol-1′-yl)-pentan-3-one (compound No. 1.6);
2-(2,4-dichlorophenyl)-2-methoxy-1-(1H-1,2,4-triazol-1′-yl)-butan-3-one (compound No. 2.2);
2-(2-chloro-4-fluorophenyl)-2-hydroxy-1-(1H-1,2,4-triazol-1′-yl)-butan-3-one (compound No. 1.16);
2-[2-methyl-4-(4′-chlorophenoxy)]-2-hydroxy-1-(1H-1,2,4-triazol-1′-yl)-butan-3-one (compound No. 1.36).

7. A process for the preparation of the compounds of formula I defined in claim 1, which comprises either reacting an oxirane of the formula II

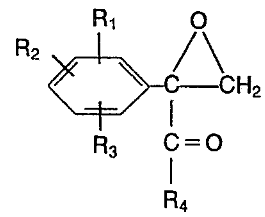

(II)

with an azole of the formula III

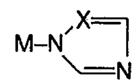

(III)

to give first a compound of the formula Ia

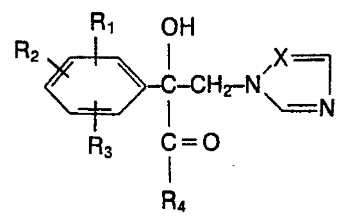

(Ia) ,

or reacting an α-haloketone or an α-haloaldehyde of the formula IV

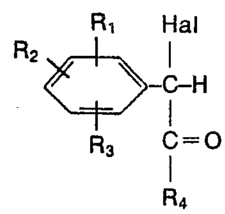

(IV)

with paraformaldehyde and a strong non-nucleophilic base and, by subsequent addition of an azole of the formula III, further reacting the intermediate to give a corresponding alcohol of the formula Ia and, in both variants, if desired converting the alcohol Ia in conventional manner, for example by reaction with a compound of the formula V

$$R_5\text{–}W \qquad (V)$$

into an ether of the formula I, in which formulae Ia, II, III, IV and V the substituents $R_1$, $R_2$, $R_3$, $R_4$, $R_5$ and X are as defined for formula I, M is hydrogen or a metal atom, Hal is halogen, and W is OH or a customary leaving group.

8. A composition for controlling or preventing attack by microorganisms and/or for regulating plant growth, which composition contains as at least one active component a compound of the formula I according to any one of claims 1 to 6, together with customary adjuvants.

9. A method of controlling or preventing attack of cultivated plants by phytopathogenic microorganisms, and/or of regulating plant growth, which

comprises applying a compound of the formula I as defined in chlaims 1 to 6 to the plants or to the locus thereof.

10. The use of compounds of the formula I according to claim 1 for the control and/or prevention of an attack by microorganisms.

11. The oxiranes of the formula II

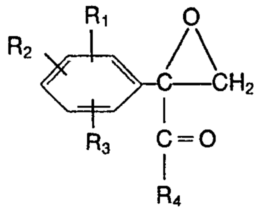

(II)

wherein $R_1$ to $R_4$ are as defined for formula I, with the exception of the compounds 2-formyl-2-phenyl-oxirane and 2-benzoyl-2-phenyl-oxirane.

**Claims for the contracting state AT**

1. A microbicidal composition which, in addition to customary adjuvants, contains as active component at least one compound of the formula I

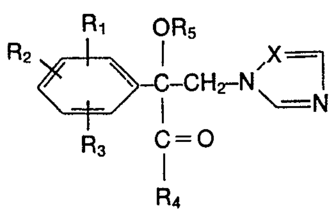

(I)

wherein

$R_1$, $R_2$ and $R_3$ are each independently hydrogen, halogen, $C_1$–$C_4$-alkyl, $CF_3$, phenyl, phenyl substituted by $C_1$–$C_4$-alkyl, $C_1$–$C_4$-alkoxy, $C_1$–$C_3$-haloalkyl, halogen and/or by cyano, phenoxy or phenoxy substituted by $C_1$–$C_4$-alkyl, $C_1$–$C_4$-alkoxy, $C_1$–$C_3$-haloalkyl, halogen and/or by cyano;

$R_4$ is hydrogen, $C_1$–$C_{10}$-alkyl, $C_3$–$C_6$-cycloalkyl, phenyl or phenyl substituted by $C_1$–$C_4$-alkyl, $C_1$–$C_4$-alkoxy, $C_1$–$C_3$-haloalkyl, halogen and/or by cyano;

X is –CH= or –N=; and

$R_5$ is hydrogen, $C_1$–$C_{12}$-alkyl, $C_2$–$C_4$-alkenyl, $C_2$–$C_4$-alkynyl, benzyl or benzyl substituted by $C_1$–$C_4$-alkyl, $C_1$–$C_4$-alkoxy, $C_1$–$C_3$-haloalkyl, halogen and/ or by cyano; including the acid addition salts, quaternary azolium salts and metal complexes thereof.

2. A composition according to claim 1, containing as active component at least one compound of the formula I wherein $R_1$, $R_2$ and $R_3$ are each independently hydrogen, halogen, $C_1$–$C_3$-alkyl, $CF_3$, phenyl, phenoxy or phenyl or phenoxy substituted by $C_1$–$C_4$-alkyl, $C_1$–$C_4$-alkoxy, $C_1$–$C_3$-haloalkyl, halogen or by cyano; $R_4$ is hydrogen, $C_1$–$C_6$-alkyl, $C_3$–$C_6$-cycloalkyl, phenyl or phenyl substituted by $C_1$–$C_4$-alkyl, $C_1$–$C_4$-alkoxy, $C_1$–$C_3$-haloalkyl, halogen or by cyano; $R_5$ is hydrogen, $C_1$–$C_8$-alkyl, $C_2$–$C_4$-alkenyl, propargyl, benzyl or benzyl mono- or di-substituted by fluorine, chlorine, bromine and/or by $C_1$–$C_3$-alkyl, and X is –CH= or –N=; including the acid addition salts, quaternary azolium salts and metal complexes thereof.

3. A composition according to claim 2, containing as active component at least one compound of the formula I wherein $R_1$, $R_2$ and $R_3$ are each independently hydrogen, fluorine, chlorine, bromine, methyl, ethyl, $CF_3$, phenyl, phenoxy, $C_6H_4Cl(4)$, $C_6H_4CH_3(4)$, $C_6H_3Cl_2(2,4)$, $OC_6H_4Cl(4)$, $OC_6H_4CH_3(4)$ or $OC_6H_3Cl_2(2,4)$; $R_4$ is hydrogen, $C_1$–$C_4$-alkyl, $C_3$–$C_6$-cycloalkyl, phenyl or phenyl substituted by methyl, methoxy, $CF_3$, F, Cl, Br or by CN; $R_5$ is hydrogen, $C_1$–$C_6$-alkyl, $C_3$–$C_4$-alkenyl, propargyl, benzyl or benzyl mono- or di-substituted by fluorine, chlorine and/or by methyl; and X is –N=; including the acid addition salts, quaternary azolium salts and metal complexes thereof.

4. A composition according to claim 3, containing as active component at least one compound of the formula I wherein $R_1$ is hydrogen, 2-Cl, 2-Br, 2-F, 2-$CF_3$, 2-$C_6H_5$, 2-$OC_6H_5$, 3-F, 3-Cl, 3-Br, 3-$CF_3$, 3-$C_6H_5$ or 3-$OC_6H_5$; $R_2$ is hydrogen, 4-Cl, 4-Br, 4-F, 4-$CF_3$, 4-$C_6H_5$, 4-$OC_6H_5$, 4-$C_6H_4Cl(4)$, 4-$C_6H_4CH_3(4)$, 4-$C_6H_3Cl_2(2,4)$, 4-$OC_6H_4Cl(4)$, 4-$OC_6H_4CH_3(4)$, 4-$OC_6H_3Cl_2(2,4)$, 4-O-$C_6H_4F(4)$, 5-Cl, 5-Br, 5-F or 5-$CF_3$; $R_3$ is hydrogen; $R_4$ is hydrogen, methyl, phenyl or 2,4-dichlorophenyl; $R_5$ is hydrogen, $C_1$–$C_5$-alkyl, allyl, propargyl, benzyl, 2-halobenzyl, 4-halobenzyl, 2,4-dihalobenzyl, 2,6-dihalobenzyl or 3,4-dihalobenzyl and X is –N=; including the acid addition salts, quaternary azolium salts and metal complexes thereof.

5. A composition according to claim 4, containing as active component at least one compound of the formula I wherein $R_1$ is 2-H, 2-F, 2-Cl or 2-Br; $R_2$ is 4-F, 4-Cl, 4-Br or 4-$CF_3$; $R_3$ is hydrogen; $R_4$ is hydrogen, methyl, phenyl or 2,4-dichlorophenyl; $R_5$ is hydrogen, $C_1$–$C_5$-alkyl, allyl, propargyl, benzyl, 2,6-dichlorobenzyl, 4-chlorobenzyl, 4-fluorobenzyl or 2,4-dichlorobenzyl; and X is –N=; including the acid addition salts, quaternary azolium salts and metal complexes thereof.

6. A composition according to claim 1, containing as active component at least one compound of the formula I selected from the group consisting of:

2-(2,4-dichlorophenyl)-2-hydroxy-3-(1H-1,2,4-triazol-1'-yl)-propanal (compound No. 1.54);

2-(2,4-dichlorophenyl)-2-hydroxy-1-(1H-1,2,4-triazol-1'-yl)-butan-3-one (compound No. 1.2);

2-(2,4-dichlorophenyl)-2-hydroxy-1-(1H-1,2,4-triazol-1'-yl)-pentan-3-one (compound No. 1.6);

2-(2,4-dichlorophenyl)-2-methoxy-1-(1H-1,2,4-triazol-1'-yl)-butan-3-one (compound No. 2.2);

2-(2-chloro-4-fluorophenyl)-2-hydroxy-1-(1H-1,2,4-triazol-1'-yl)-butan-3-one (compound No. 1.16);

2-[2-methyl-4-(4'-chlorophenoxy)]-2-hydroxy-1-(1H-1,2,4-triazol-1'-yl)-butan-3-one (compound No. 1.36).

7. A process for the preparation of the compounds of formula I defined in claim 1, which comprises either reacting an oxirane of the formula II

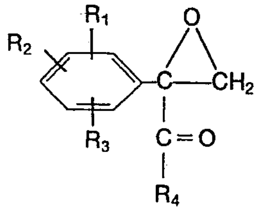

(II)

with an azole of the formula III

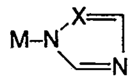

(III)

to give first a compound of the formula Ia

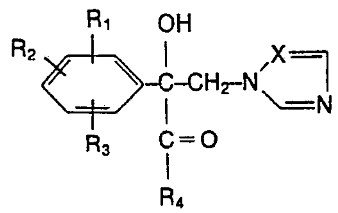

(Ia) ,

or reacting an α-haloketone or an α-haloaldehyde of the formula IV

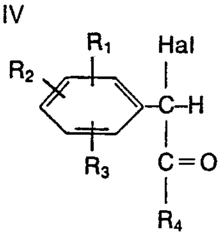

(IV)

with paraformaldehyde and a strong non-nucleophilic base and, by subsequent addition of an azole of the formula III, further reacting the intermediate to give a corresponding alcohol of the formula Ia and, in both variants, if desired converting the alcohol Ia in conventional manner, for example by reaction with a compound of the formula V

$$R_5\text{–}W \qquad (V)$$

into an ether of the formula I, in which formulae Ia, II, III, IV and V the substituents $R_1$, $R_2$, $R_3$, $R_4$, $R_5$ and X are as defined for formula I, M is hydrogen or a metal atom, Hal is halogen, and W is OH or a customary leaving group.

8. A process for the preparation of an agrochemical composition which comprises intimately mixing at least one compound of the formula I as defined in any one of claims 1 to 6 with suitable solid or liquid adjuvants and surfactants.

9. A method of controlling or preventing attack of cultivated plants by phytopathogenic microorganisms, and/or of regulating plant growth, which comprises applying a compound of the formula I as defined in claims 1 to 6 to the plants or to the locus thereof.

10. The use of compounds of the formula I according to claim 1 for the control and/or prevention of an attack by microorganisms.

11. The use of oxiranes of the formula II

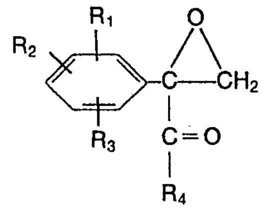

(II)

wherein $R_1$ to $R_4$ are as defined for formula I, with the exception of the compounds 2-formyl-2-phenyl-oxirane and 2-benzoyl-2-phenyl-oxirane.

**Revendications pour les états contractants: BE, CH, DE, FR, GB, IT, LI, LU, NL, SE**

1. Composés de formule I

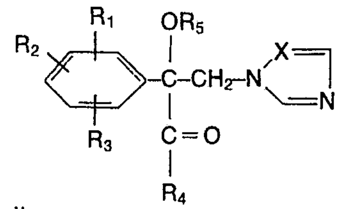

(I)

dans laquelle

$R_1$, $R_2$ and $R_3$ représentent chacun, indépendamment les uns des autres, l'hydrogène, un halogène, un groupe alkyle en $C_1$–$C_4$, $CF_3$, phényle ou phényle substitué par des groupes alkyle en $C_1$–$C_4$, alcoxy en $C_1$–$C_4$, halogénoalkyle en $C_1$–$C_3$, des halogènes et/ou des groupes cyano, ou phénoxy ou phénoxy substitué par des groupes alkyle en $C_1$–$C_4$, alcoxy en $C_1$–$C_4$, halogénoalkyle en $C_1$–$C_3$, des halogènes et/ou des groupes cyano;

$R_4$ représente l'hydrogène, un groupe alkyle en $C_1$–$C_{10}$, cycloalkyle en $C_3$–$C_6$, phényle ou phényle substitué par des groupes alkyle en $C_1$–$C_4$, alcoxy en $C_1$–$C_4$, halogénoalkyle en $C_1$–$C_3$, des halogènes et/ou des groupes cyano;

X représente –CH= ou –N=; et

$R_5$ représente l'hydrogène, un groupe alkyle en $C_1$–$C_{12}$, alcényle en $C_2$–$C_4$, alcynyle en $C_2$–$C_4$, benzyle ou benzyle substitué par des groupes alkyle en $C_1$–$C_4$, alcoxy en $C_1$–$C_4$, halogénoalkyle en $C_1$–$C_3$, des halogènes et/ou des groupes cyano; y compris leurs sels formés par addition avec des acides, leurs sels d'azolium quaternaire et leurs complexes métalliques.

2. Composés de formule I selon la revendication 1, dans laquelle $R_1$, $R_2$ et $R_3$ représentent chacun, indépendamment les uns des autres, l'hydrogène, un halogène, un groupe alkyle en $C_1$–$C_3$, $CF_3$, phényle, phénoxy ou phényle ou phénoxy substitué par des groupes alkyle en $C_1$–$C_4$, alcoxy en $C_1$–$C_4$, halogénoalkyle en $C_1$–$C_3$, des halogènes ou des groupes cyano; $R_4$ représente l'hydrogène, un groupe alkyle en $C_1$–$C_6$, cycloalkyle en $C_3$–$C_6$, phényle ou phényle substitué par des groupes alkyle en $C_1$–$C_4$, alcoxy en $C_1$–$C_4$, halogénoalkyle en $C_1$–$C_3$, des halogènes ou des groupes cyano; $R_5$ représente l'hydrogène, un groupe alkyle en $C_1$–$C_8$, alcényle en $C_2$–$C_4$, propargyle, benzyle ou benzyle mono- ou di-substitué par le fluor, le chlore, le brome et/ou des groupes alkyle en $C_1$–$C_3$ et X représente –CH= ou –N=; y compris leurs sels

formés par addition avec des acides, sels d'azolium quaternaire et complexes métalliques.

3. Composés de formule I selon la revendication 2, dans laquelle $R_1$, $R_2$ et $R_3$ représentent chacun, indépendamment les uns des autres, l'hydrogène, le fluor, le chlore, le brome, un groupe méthyle, éthyle, $CF_3$, phényle, phénoxy, $C_6H_4Cl(4)$, $C_6H_4CH_3(4)$, $C_6H_3Cl_2(2,4)$, $OC_6H_4Cl(4)$, $OC_6H_4CH_3(4)$ ou $OC_6H_3Cl_2(2,4)$; $R_4$ représente l'hydrogène, un groupe alkyle en $C_1$–$C_4$, cycloalkyle en $C_3$–$C_6$, phényle ou phényle substitué par des groupes méthyle, méthoxy, $CF_3$, F, Cl, Br ou CN; $R_5$ représente l'hydrogène, un groupe alkyle en $C_1$–$C_6$, alcényle en $C_3$–$C_4$, propargyle, benzyle ou benzyle mono- ou di-substitué par le fluor, le chlore et/ou des groupes méthyle; et X représente –N=; y compris leurs sels formés par addition avec des acides, sels d'azolium quaternaire et complexes métalliques.

4. Composés de formule I selon la revendication 3, dans laquelle $R_1$ représente l'hydrogène, 2-Cl, 2-Br, 2-F, 2-$CF_3$, 2-$C_6H_5$, 2-$OC_6H_5$, 3-F, 3-Cl, 3-Br, 3-$CF_3$, 3-$C_6H_5$ ou 3-$OC_6H_5$; $R_2$ représente l'hydrogène, 4-Cl, 4-Br, 4-F, 4-$CF_3$, 4-$C_6H_5$, 4-$OC_6H_5$, 4-$C_6H_4Cl(4)$, 4-$C_6H_4CH_3(4)$, 4-$C_6H_3Cl_2(2,4)$, 4-$OC_6H_4Cl(4)$, 4-$OC_6H_4CH_3(4)$, 4-$OC_6H_3Cl_2(2,4)$, 4-O-$C_6H_4F(4)$, 5-Cl, 5-Br, 5-F ou 5-$CF_3$; $R_3$ représente l'hydrogène; $R_4$ représente l'hydrogène, un groupe méthyle, phényle ou 2,4-dichlorophényle; $R_5$ représente l'hydrogène, un groupe alkyle en $C_1$–$C_5$, allyle, propargyle, benzyle, 2-halogénobenzyle, 4-halogénobenzyle, 2,4-dihalogénobenzyle, 2,6-dihalogénobenzyle ou 3,4-dihalogénobenzyle et X représente –N=; y compris leurs sels formés par addition avec des acides, sels d'azolium quaternaire et complexes métalliques.

5. Composés de formule I selon la revendication 4, dans laquelle $R_1$ représente 2-H, 2-F, 2-Cl ou 2-Br; $R_2$ représente 4-F, 4-Cl, 4-Br ou 4-$CF_3$; $R_3$ représente l'hydrogène;

$R_4$ représente l'hydrogène, un groupe méthyle, phényle ou 2,4-dichlorophényle;

$R_5$ représente l'hydrogène, un groupe alkyle en $C_1$–$C_5$, allyle, propargyle, benzyle, 2,6-dichlorobenzyle, 4-chlorobenzyle, 4-fluorobenzyle ou 2,4-dichlorobenzyle; et X représente –N=; y compris leurs sels formés par addition avec des acides, sels d'azolium quaternaire et complexes métalliques.

6. Un composé de formule I selon la revendication 1, choisi parmi les suivants:

2-(2,4-dichlorophényl)-2-hydroxy-3-(1H-1,2,4-triazole-1'-yl)-propanal (composé no 1.54);

2-(2,4-dichlorophényl)-2-hydroxy-1-(1H-1,2,4-triazole-1'-yl)-butane-3-one (composé no 1.2);

2-(2,4-dichlorophényl)-2-hydroxy-1-(1H-1,2,4-triazole-1'-yl)-pentane-3-one (composé no 1.6);

2-(2,4-dichlorophényl)-2-méthoxy-1-(1H-1,2,4-triazole-1'-yl)-butane-3-one (composé no 2.2);

2-(2-chloro-4-fluorophényl)-2-hydroxy-1-(1H-1,2,4-triazole-1'-yl)-butane-3-one (composé no 1.16);

2-[2-méthyl-4-(4'-chlorophénoxy)]-2-hydroxy-1-(1H-1,2,4-triazole-1'-yl)-butane-3-one (composé no 1.36).

7. Procédé de préparation des composés de formule I définie dans la revendication 1, caractérisé en ce que:

soit on fait d'abord réagir un oxiranne de formule II

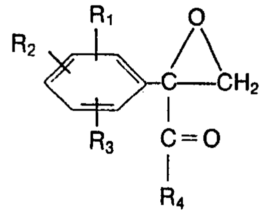

(II)

avec un azole de formule III

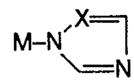

(III)

la réaction donnant un composé de formule Ia

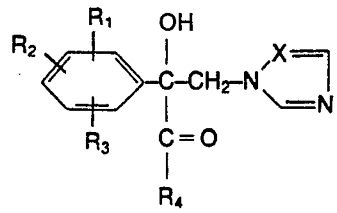

(Ia)

soit on fait réagir une alpha-halogénocétone ou un alpha-halogénoaldéhyde de formule IV

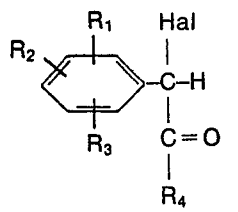

(IV)

avec le paraformaldéhyde et une base forte non nucléophile et, en ajoutant ensuite un azole de formule III, on poursuit la réaction jusqu'à formation d'un alcool correspondant de formule Ia, et dans les deux cas, si on le désire, on convertit l'alcool Ia de la manière habituelle, par exemple par réaction avec un composé de formule V

$$R_5\text{–}W \qquad (V)$$

en un éther de formule I, les symboles $R_1$, $R_2$, $R_3$, $R_4$, $R_5$ et X ayant, dans les formules Ia, II, III, IV et V, les significations indiquées en référence à la formule I, M représentant l'hydrogène ou un atome métallique, Hal un halogène et W un groupe OH ou un groupe éliminable usuel.

8. Produit pour prévenir ou combattre une infestation par des microorganismes et/ou pour la régulation de la croissance des végétaux, caractérisé en ce qu'il contient au moins un composant actif consistant en un composé de formule I selon l'une des revendications 1 à 6, avec des additifs usuels.

9. Procédé pour prévenir ou combattre une infestation de végétaux cultivés par des microorganismes phytopathogènes et/ou pour la régulation de la croissance des végétaux, caractérisé en ce que l'on applique sur les plantes ou leur habitat un composé de formule I définie dans les revendications 1 à 6.

10. Utilisation des composés de formule I selon la revendication 1, pour combattre et/ou prévenir une infestation par des microorganismes.

11. Les oxirannes de formule II

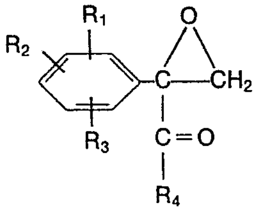

(II)

dans laquelle $R_1$ à $R_4$ ont les significations indiquées en référence à la formule I, à l'exception des composés 2-formyl-2-phényl-oxiranne et 2-benzoyl-2-phényl-oxiranne.

**Revendications pour l'état contractant AT**

1. Produit microbicide caractérisé en ce qu'il contient, avec des additifs usuels, à titre de composant actif au moins un composé de formule I

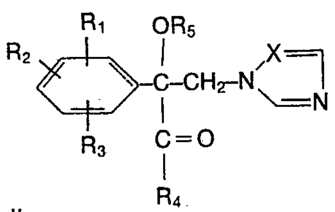

(I)

dans laquelle

$R_1$, $R_2$ et $R_3$ représentent chacun, indépendamment les uns des autres, l'hydrogène, un halogène, un groupe alkyle en $C_1$–$C_4$, $CF_3$, phényle ou phényle substitué par des groupes alkyle en $C_1$–$C_4$, alcoxy en $C_1$–$C_4$, halogénoalkyle en $C_1$–$C_3$, des halogènes et/ou des groupes cyano, ou phénoxy ou phénoxy substitué par des groupes alkyle en $C_1$–$C_4$, alcoxy en $C_1$–$C_4$, halogénoalkyle en $C_1$–$C_3$, des halogènes et/ou des groupes cyano;

$R_4$ représente l'hydrogène, un groupe alkyle en $C_1$–$C_{10}$, cycloalkyle en $C_3$–$C_6$, phényle ou phényle substitué par des groupes alkyle en $C_1$–$C_4$, alcoxy en $C_1$–$C_4$, halogénoalkyle en $C_1$–$C_3$, des halogènes et/ou des groupes cyano;

X représente –CH= ou –N=; et

$R_5$ représente l'hydrogène, un groupe alkyle en $C_1$–$C_{12}$, alcényle en $C_2$–$C_4$, alcynyle en $C_2$–$C_4$, benzyle ou benzyle substitué par des groupes alkyle en $C_1$–$C_4$, alcoxy en $C_1$–$C_4$, halogénoalkyle en $C_1$–$C_3$, des halogènes et/ou des groupes cyano; y compris leurs sels formés par addition avec des acides, leurs sels d'azolium quaternaire et leurs complexes métalliques.

2. Produit selon la revendication 1, contenant au moins à titre de composant actif au moins un composé de formule I dans laquelle $R_1$, $R_2$ et $R_3$ représentent chacun, indépendamment les uns des autres, l'hydrogène, un halogène, un groupe alkyle en $C_1$–$C_3$, $CF_3$, phényle, phénoxy ou phényle ou phénoxy substitué par des groupes alkyle en $C_1$–$C_4$, alcoxy en $C_1$–$C_4$, halogénoalkyle en $C_1$–$C_3$, des halogènes ou des groupes cyano; $R_4$ représente l'hydrogène, un groupe alkyle en $C_1$–$C_6$, cycloalkyle en $C_3$–$C_6$, phényle ou phényle substitué par des groupes alkyle en $C_1$–$C_4$, alcoxy en $C_1$–$C_4$, halogénoalkyle en $C_1$–$C_3$, des halogènes ou des groupes cyano; $R_5$ représente l'hydrogène, un groupe alkyle en $C_1$–$C_8$, alcényle en $C_2$–$C_4$, propargyle, benzyle ou benzyle mono- ou di-substitué par le fluor, le chlore, le brome et/ou des groupes alkyle en $C_1$–$C_3$ et X représente –CH= ou –N=; y compris leurs sels formés par addition avec des acides, sels d'azolium quaternaire et complexes métalliques.

3. Produit selon la revendication 2, contenant à titre de composant actif au moins un composé de formule I dans laquelle $R_1$, $R_2$ et $R_3$ représentent chacun, indépendamment les uns des autres, l'hydrogène, le fluor, le chlore, le brome, un groupe méthyle, éthyle, $CF_3$, phényle, phénoxy, $C_6H_4Cl(4)$, $C_6H_4CH_3(4)$, $C_6H_3Cl_2(2,4)$, $OC_6H_4Cl(4)$, $OC_6H_4CH_3(4)$ ou $OC_6H_3Cl_2(2,4)$; $R_4$ représente l'hydrogène, un groupe alkyle en $C_1$–$C_4$, cycloalkyle en $C_3$–$C_6$, phényle ou phényle substitué par des groupes méthyle, méthoxy, $CF_3$, F, Cl, Br ou CN; $R_5$ représente l'hydrogène, un groupe alkyle en $C_1$–$C_6$, alcényle en $C_3$–$C_4$, propargyle, benzyle ou benzyle mono- ou di-substitué par le fluor, le chlore et/ou des groupes méthyle; et X représente –N=; y compris leurs sels formés par addition avec des acides, sels d'azolium quaternaires et complexes métalliques.

4. Produit selon la revendication 3, contenant à titre de composant actif au moins un composé de formule I dans laquelle $R_1$ représente l'hydrogène, 2-Cl, 2-Br, 2-F, 2-$CF_3$, 2-$C_6H_5$, 2-$OC_6H_5$, 3-F, 3-Cl, 3-Br, 3-$CF_3$, 3-$C_6H_5$ ou 3-$OC_6H_5$; $R_2$ représente l'hydrogène, 4-Cl, 4-Br, 4-F, 4-$CF_3$, 4-$C_6H_5$, 4-$OC_6H_5$, 4-$C_6H_4Cl(4)$, 4-$C_6H_4CH_3(4)$, 4-$C_6H_3Cl_2(2,4)$, 4-$OC_6H_4Cl(4)$, 4-$OC_6H_4CH_3(4)$, 4-$OC_6H_3Cl_2(2,4)$, 4-O-$C_6H_4F(4)$, 5-Cl, 5-Br, 5-F ou 5-$CF_3$; $R_3$ représente l'hydrogène; $R_4$ représente l'hydrogène, un groupe méthyle, phényle ou 2,4-dichlorophényle; $R_5$ représente l'hydrogène, un groupe alkyle en $C_1$–$C_5$, allyle, propargyle, benzyle, 2-halogénobenzyle, 4-halogénobenzyle, 2,4-dihalogénobenzyle, 2,6-dihalogénobenzyle ou 3,4-dihalogénobenzyle et X représente –N=; y compris leurs sels formés par addition avec des acides, sels d'azolium quaternaire et complexes métalliques.

5. Produit selon la revendication 4, contenant à titre de composant actif au moins un composé de formule I dans laquelle $R_1$ représente 2-H, 2-F, 2-Cl ou 2-Br; $R_2$ représente 4-F, 4-Cl, 4-Br ou 4-$CF_3$; $R_3$ représente l'hydrogène;

$R_4$ représente l'hydrogène, un groupe méthyle, phényle ou 2,4-dichlorophényle,

$R_5$ représente l'hydrogène, un groupe alkyle en $C_1$–$C_5$, allyle, propargyle, benzyle, 2,6-dichlorobenzyle, 4-chlorobenzyle, 4-fluorobenzyle ou 2,4-dichlorobenzyle; et X représente –N=; y compris

leurs sels formés par addition avec des acides, sels d'azolium quaternaire et complexes métalliques.

6. Produit selon la revendication 1, contenant à titre de composant actif au moins un composé de formule I choisi parmi les suivants:

2-(2,4-dichlorophényl)-2-hydroxy-3-(1H-1,2,4-(triazole-1'-yl)-propanal (composé no 1.54);

2-(2,4-dichlorophényl)-2-hydroxy-1-(1H-1,2,4-(triazole-1'-yl)-butane-3-one (composé no 1.2);

2-(2,4-dichlorophényl)-2-hydroxy-1-(1H-1,2,4-(triazole-1'-yl)-pentane-3-one (composé no 1.6);

2-(2,4-dichlorophényl)-2-méthoxy-1-(1H-1,2,4-(triazole-1'-yl)-butane-3-one (composé no 2.2);

2-(2-chloro-4-fluorophényl)-2-hydroxy-1-(1H-1,2,4-triazole-1'-yl)-butane-3-one (composé no. 1.16);

2-[2-méthyl-4-(4'-chlorophénoxy)]-2-hydroxy-1-(1H-1,2,4-triazole-1'-yl)-butane-3-one (composé no 1.36).

7. Procédé de préparation des composés de formule I définis dans la revendication 1, caractérisé en ce que:

on fait d'abord réagir un oxiranne de formule II

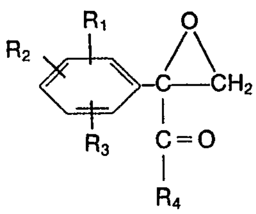

(II)

avec un azole de formule III

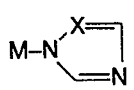

(III)

la réaction donnant un composé de formule Ia

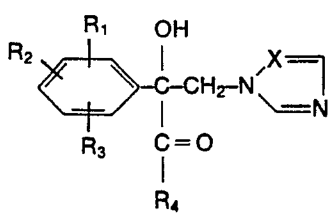

(Ia)

ou bien on fait réagir une alpha-halogénocétone ou un alpha-halogénoaldéhyde de formule IV

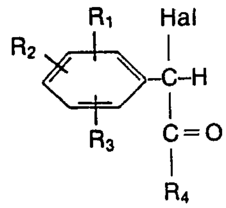

(IV)

avec le paraformaldéhyde et une base forte non nucléophile et, en ajoutant ensuite un azole de formule III, on poursuit la réaction jusqu'à formation d'un alcool correspondant de formule Ia, et dans les deux cas, si on le désire, on convertit l'alcool Ia de la manière habituelle, par exemple par réaction avec un composé de formule V

$$R_5\text{–}W \qquad (V)$$

en un éther de formule I, les symboles $R_1$, $R_2$, $R_3$, $R_4$, $R_5$ et X ayant, dans les formules Ia, II, III, IV et V, les significations indiquées en référence à la formule I, M représentant l'hydrogène ou un atome métallique, Hal un halogène et W un groupe OH ou un groupe éliminable usuel.

8. Procédé de préparation d'un produit agrochimique, caractérisé en ce que l'on mélange intimement au moins un composé de formule I défini dans l'une des revendications 1 à 6 avec des additifs solides ou liquides appropriés et des agents tensioactifs.

9. Procédé pour prévenir ou combattre une infestation de végétaux cultivés par des microorganismes phytopathogènes et/ou pour la régulation de la croissance des végétaux, caractérisé en ce que l'on applique sur les végétaux ou leur habitat un composé de formule I définie dans les revendications 1 à 6.

10. Utilisation des composés de formule I selon la revendication 1, pour combattre et/ou prévenir une infestation par des microorganismes.

11. Utilisation des oxirannes de formule II

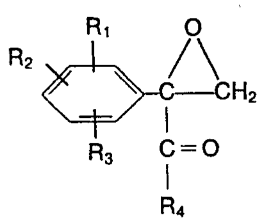

(II)

dans laquelle $R_1$ à $R_4$ ont les significations indiquées en référence à la formule I, à l'exception des composés 2-formyl-2-phényl-oxiranne et 2-benzoyl-2-phényl-oxiranne.